# FASCICULE DE BREVET EUROPEEN

(11) **EP 2 040 703 B1**
(45) Date de publication et mention de la délivrance du brevet: **21.10.2009**
(21) Numéro de dépôt: 07803831.2
(22) Date de dépôt: 03.07.2007
(51) Int. Cl.: A61K 31/4188, A61K 31/4353, C07D 471/04, A61P 25/14

(54) **DERIVES DE 2-BENZOYL-IMIDAZOPYRIDINES, LEUR PREPARATION ET LEUR APPLICATION EN THERAPEUTIQUE**
2-BENZOYL-IMIDAZOPYRIDIN-DERIVATE, HERSTELLUNGSVERFAHREN UND IHRE VERWENDUNG FÜR THERAPEUTIKA
DERIVATIVES OF 2-BENZOYL-IMIDAZOPYRIDINES, PREPARATION METHOD THEREOF AND USE OF SAME IN THERAPEUTICS

(30) Priorité: 03.07.2006 FR 0606010
(43) Date de publication de la demande: 01.04.2009
(73) Titulaire: Sanofi-Aventis, 75013 Paris (FR)
(72) Inventeur: EL-AHMAD, Youssef, 92160 Antony (FR); PEYRONEL, Jean-François, 92160 Antony (FR)
(74) Mandataire: Morel-Pécheux, Muriel
(86) Numéro de dépôt international: PCT/FR2007/001123
(87) Numéro de publication internationale: WO 2008/003854

(56) Documents cités:
- WO-A-03/059884
- WO-A2-01/74813
- WO-A2-20/05044793
- FR-A1- 2 638 161
- US-A1- 2004 204 409
- THEUNS J ET AL: "O4-06-03 A novel NR4A2 promoter variation associated with Parkinson's disease alters gene expression" NEUROBIOLOGY OF AGING, TARRYTOWN, NY, US, vol. 25, juillet 2004 (2004-07), page S85, XP004624904 ISSN: 0197-4580

## Description

La présente invention se rapporte à des dérivés de 2-benzoyl-imidazo[1,2-*a*]pyridine, à leur préparation et à leur application en thérapeutique dans le traitement ou la prévention de maladies impliquant les récepteurs nucléaires Nurr-1 aussi appelés NR4A2, NOT, TINUR, RNR-1, et HZF3.

La présente invention a pour objet les composés répondant à la formule (I) : 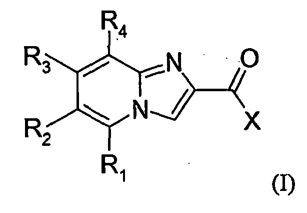 dans laquelle :
X représente:
   . un groupe phényle éventuellement substitué par un ou plusieurs atomes ou groupes choisis indépendamment les uns des autres parmi les atomes ou groupes suivants : halogène, (C₁-C₆)alcoxy, (C₁-C₆)alkyle, (C₃-C₇)cycloalkyle(C₁-C₆)alkyle, (C₃-C₇)cycloalkyle(C₁-C₆)alcoxy, hydroxy, amino, NRaRb ;
R₁ représente un atome d'hydrogène, un halogène, un groupe (C₁-C₆)alcoxy, un groupe (C₁-C₆)alkyle, un groupe (C₃-C₇)cycloalkyle(C₁-C₆)alkyle, un groupe (C₃-C₇)cycloalkyle(C₁-C₆)alcoxy, un hydroxy, un amino; le groupe (C₁-C₆)alkyle pouvant éventuellement être substitué par un ou plusieurs atomes ou groupes halogènes, hydroxy, amino, (C₁-C₆)alcoxy et le groupe (C₁-C₆)alcoxy pouvant éventuellement être substitué par un ou plusieurs atomes ou groupes halogène, hydroxy, amino, (C₁-C₆)alcoxy;
R₂ représente l'un des groupes suivants :
   - un atome d'hydrogène,
   - un groupe (C₁-C₆)alkyle éventuellement substitué par un ou plusieurs atomes ou groupes choisis indépendamment les uns des autres parmi halogène, hydroxy, amino, NRaRb,
   - un groupe (C₁-C₆)alcoxy substitué par un ou plusieurs atomes ou groupes choisis indépendamment les uns des autres parmi halogène, hydroxy, amino, NRaRb,
   - un groupe (C₃-C₇)cydoalkyle(C₁-C₆)alkyle,
   - un groupe (C₃-C₇)cycloalkyle(C₁-C₆)alcoxy,
   - un groupe (C₂-C₆)alcényle,
   - un groupe (C₂-C₆)alcynyle,
   - un groupe -CO-R₅
   - un groupe -CO-NR₆R₇
   - un groupe -CO-O-R₈
   - un groupe NR₉-CO-R₁₀
   - un groupe -NR₁₁R₁₂
   - un atome d'halogène,
   - un groupe cyano,
   - un groupe phényle éventuellement substitué par un ou plusieurs groupes choisis indépendamment les uns des autres parmi les atomes ou groupes halogène, (C₁-C₆)alcoxy, (C₁-C₆)alkyle, (C₃-C₇)cycloalkyle(C₁-C₆)alkyle, (C₃-C₇)cycloalkyle(C₁-C₆)alcoxy, hydroxy, amino, NRaRb, CO-R₅, le groupe (C₁-C₆)alkyle étant éventuellement substitué par un groupe hydroxy,
R₃ représente un atome d'hydrogène, un groupe (C₁-C₆)alkyle, un atome d'halogène, ou un groupe hydroxy,
R₄ représente un atome d'hydrogène ou un atome d'halogène,
R₅ représente un atome d'hydrogène ou un groupe (C₁-C₆)alkyle,
R₆ et R₇, identiques ou différents, représentent un atome d'hydrogène ou un groupe (C₁-C₆)alkyle ou forment avec l'atome d'azote un cycle de 4 à 7 chaînons incluant éventuellement un autre hétéroatome choisi parmi N, O ou S,
R₈ représente un groupe (C₁-C₆)alkyle,
R₉ et R₁₀, identiques ou différents, représentent un atome d'hydrogène ou un groupe (C₁-C₆)alkyle, R₁₁ représente un groupe (C₁-C₆)alkyle,
R₁₂ représente un hydrogène ou un groupe (C₁-C₆)alkyle,
R₁₁ et R₁₂ peuvent former avec l'atome d'azote un cycle de 4 à 7 chaînons incluant éventuellement un autre hétéroatome choisi parmi N, O ou S,
Ra représente un (C₁-C₆)alkyle
Rb représente un hydrogène ou un (C₁-C₆)alkyle
   l'un au moins des substituants R₁, R₂, R₃ et R₄ n'est pas un hydrogène
   à l'exception du composé où X est un phényle, R₃ est méthyle et R₁, R₂ et R₄ sont des hydrogènes ; du composé où X est un phényle, R₂ est chlore ou méthoxy et R₁, R₃ et R₄ sont des hydrogènes ; du composé où X est un p-tolyle, R₂ est un méthyle et R₁, R₃ et R₄ sont des hydrogènes ; du composé où X est un p-chlorophényle, R₁ est un chlore ou un méthoxy ou un méthyle, et R₂, R₃ et R₄ sont des hydrogènes ; du composé où X est un p-chlorophényle, R₂ est un chlore et R₁, R₃ et R₄ sont des hydrogènes ; du composé où X est un p-chlorophényle, R₂ est un méthyle et R₁, R₃ et R₄ sont des hydrogènes ; du composé où X est un p-chlorophényle, R₄ est un méthyle, et R₁, R₂ et R₃ sont des hydrogènes ; du composé où X est un p-chlorophényle, R₃ est un méthyle et R₁, R₂ et R₄ sont des hydrogènes ; et du composé où X est un p-chlorophényle, R₁ et R₃ sont des méthyles et R₂ et R₄ sont des hydrogènes,
   à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Parmi les composés de formule (I) objets de l'invention, un premier groupe de composés est constitué des composés pour lesquels :
R₁, R₃ et R₄ sont des atomes d'hydrogène
   à l'état de base ou de sel d'addition à un acide.

Parmi les composés de formule (I) objets de l'invention, un second groupe de composés est constitué des composés pour lesquels X est un groupe phényle, à l'état de base ou de sel d'addition à un acide.

Les composés de formule (I) peuvent exister à l'état de bases ou de sels d'addition à des acides. De tels sels d'addition font partie de l'invention.
Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un atome d'halogène : un fluor, un chlore, un brome ou un iode ;
- un groupe (C₁-C₆)alkyle : un groupe aliphatique de 1 à 6 carbones saturé, linéaire, ramifié ou cyclique. A titre d'exemples, on peut citer les groupes méthyle, éthyle, propyle, isopropyle, butyle, isobutyle, tertbutyle, cyclopropyle, cyclobutyle, cyclopentyle, cyclohexyle etc ;
- un groupe (C₂-C₆)alcényle : un groupe aliphatique de 2 à 6 carbones mono- ou poly-insaturé, linéaire ou ramifié, comprenant par exemple une ou deux insaturations éthyléniques ;
- un groupe (C₁-C₆)alcoxy : un radical -O-alkyle où le groupe alkyle est tel que précédemment défini ;
- un groupe (C₂-C₆)alcynyle : un groupe aliphatique de 2 à 6 carbones mono- ou poly-insaturé, linéaire ou ramifié, comprenant par exemple une ou deux insaturations acétylèniques ;

Parmi les composés de formule (I) objets de l'invention, on peut notamment citer les composés suivants :
(6-Méthylimidazo[1,2-α]pyridin-2-yl)(phényl)méthanone et son chlorhydrate (1:1)
Phényl[6-(trifluorométhyl)imidazo[1,2-α]pyridin-2-yl]méthanone chlorhydrate (1:1)
Phényl[6-(trifluorométhyl)imidazo[1,2-α]pyridin-2-yl]méthanone
Phényl[6-(trifluorométhyl)imidazo[1,2-*a*]pyridin-2-yl]méthanone bromhydrate (1 : 1)
[6-(1-hydroxy-1-méthyléthyl)imidazo[1,2-*a*]pyridin-2-yl](phényl)méthanone et son bromhydrate (1 : 1)
[6-(Hydroxyméthyl)imidazo[1,2-α]pyridin-2-yl](phényl)méthanone
(6-Chloroimidazo[1,2-α]pyridin-2-yl)(2-méthylphényl)méthanone
Phényl(6-phénylimidazo[1,2-α]pyridin-2-yl)méthanone
[5-(2-Méthoxyéthoxy)imidazo[1,2-α]pyridin-2-yl](phényl)méthanone
*N*-(2-Benzoylimidazo[1,2-α]pyridin-6-yl)acétamide
(6-Isopropénylimidazo[1,2-α]pyridin-2-yl)(phényl)méthanone
2-Benzoyl-*N,N*-diméthylimidazo[1,2-α]pyridine-6-carboxamide
2-Benzoyl-*N*-méthylimidazo[1,2-α]pyridine-6-carboxamide
Phényl(6-vinylimidazo[1,2-α]pyridin-2-yl)méthanone
(6-Ethylimidazo[1,2-α]pyridin-2-yl)(phényl)méthanone
(6-Fluoroimidazo[1,2-α]pyridin-2-yl)(phényl)méthanone
(6,8-dichloroimidazo[1,2-α]pyridin-2-yl)(phényl)méthanone et son chlorhydrate (1:1)
(7-Chloroimidazo[1,2-α]pyridin-2-yl)(phényl)méthanone
(6-Bromoimidazo[1,2-α]pyridin-2-yl)(phényl)méthanone
(5-Bromoimidazo[1,2-α]pyridin-2-yl)(phényl)méthanone
2-Benzoylimidazo[1,2-α]pyridine-6-carbonitrile
(5-Aminoimidazo[1,2-α]pyridin-2-yl)(phényl)méthanone
2-Benzoylimidazo[1,2-α]pyridine-6-carboxamide
(6-iodolimidazo[1,2-α]pyridin-2-yl)(phényl)méthanone et son bromhydrate (1 :1)
(7-hydroxyimidazo[1,2-α]pyridin-2-yl)(phényl)méthanone et son bromhydrate (1 :1)
(6,8-difluoroimidazo[1,2-α]pyridin-2-yl)(phényl)méthanone et son bromhydrate (1 :1)
2-benzoylimidazo[1,2-α]pyridine-6-carboxylate de méthyle et son bromhydrate (1 :1)
[6-(1-Ethoxypropyl)imidazo[1,2-α]pyridin-2-yl](phényl)méthanone
1-(2-Benzoylimidazo[1,2-α]pyridin-6-yl)éthanone
2-Benzoylimidazo[1,2-α]pyridine-6-carbaldéhyde
(6-Chloroimidazo[1,2-α]pyridin-2-yl)(3-méthylphényl)méthanone
(6-Chloroimidazo[1,2-α]pyridin-2-yl)(4-méthylphényl)méthanone
(6-Chloroimidazo[1,2-α]pyridin-2-yl)(3-fluorophényl)méthanone
(6-Chloroimidazo[1,2-α]pyridin-2-yl)(4-fluorophényl)méthanone
(6-Chloroimidazo[1,2-α]pyridin-2-yl)(3,5-difluorophényl)méthanone
(5-Méthylimidazo[1,2-α]pyridin-2-yl)(phényl)méthanone
(6-Bromo-5-méthylimidazo[1,2-α]pyridin-2-yl)(phényl)méthanone
(6-Chloroimidazo[1,2-α]pyridin-2-yl)(3-chlorophényl)méthanone
(6-Chloroimidazo[1,2-α]pyridin-2-yl)(3,4-difluorophényl)méthanone
[6-(diméthylamino)imidazo[1,2-α]pyridin-2-yl](phényl)méthanone et son hexaflorophosphate (1: 1)
{6-[3-(Hydroxyméthyl)phényl]imidazo[1,2-α]pyridin-2-yl}(phényl)méthanone
{6-[4-(Hydroxyméthyl)phényl]imidazo[1,2-α]pyridin-2-yl}(phényl)méthanone
{6-[2-(Hydroxyméthyl)phényl]imidazo[1,2-α]pyridin-2-yl}(phényl)méthanone
3-(2-Benzoylimidazo[1,2-α]pyridin-6-yl)benzaldéhyde
(5,6-Diméthylimidazo[1,2-α]pyridin-2-yl)(phényl)méthanone.

Conformément à l'invention, on peut préparer les composés de formule générale (I) selon le procédé décrit dans le schéma 1.

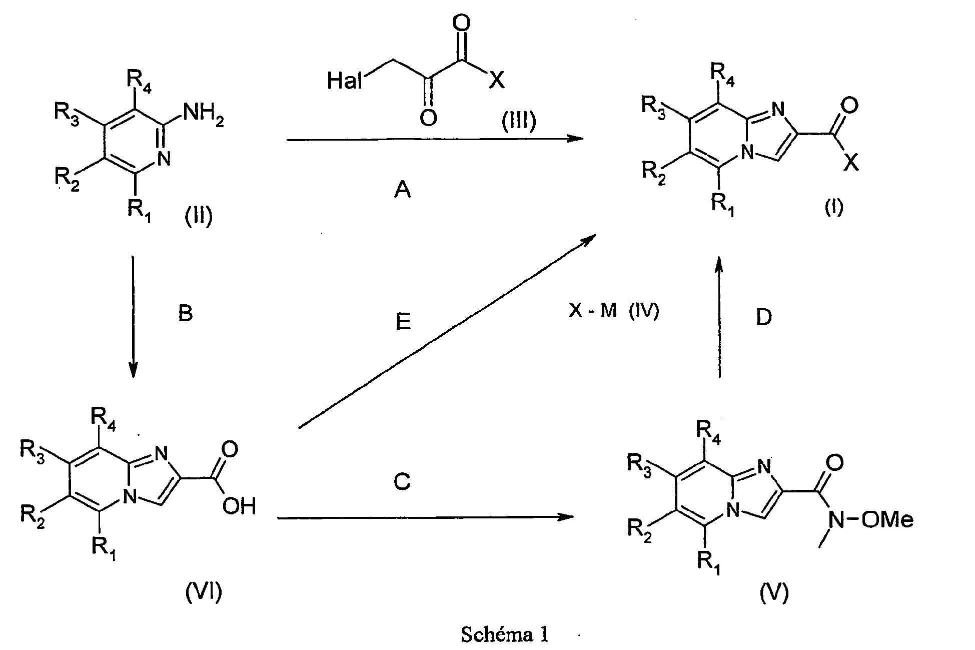

La voie A consiste à préparer les 2-amino-pyridines de formule (II) selon les méthodes connues de l'homme du métier et à former le cycle imidazo[1,2-α]pyridine par condensation sur un dérivé de 1-aryl-propane-1,2-dione (III) dans lequel Hal représente un halogène par exemple selon la méthode décrite par J-J. Bourguignon et coll. dans Aust. J. Chem.1997, 50, 719-725.

La seconde voie de synthèse B, C, D consiste à faire réagir un dérivé organométallique de formule générale (IV) dans laquelle X est défini comme ci-dessus et M représente un atome de lithium ou un groupe Mg-Hal sur un amide de Weinreb de formule (V) dont les fonctions réactives sont éventuellement protégées, selon des méthodes connues de l'homme du métier telles que décrites dans Nahm, S.; Weinreb, S. M., Tetrahedron Letters (1981), 22(39), 3815-18 et dans Sibi, M.P. Organic Preparations and Procedures Int. 1993, 25, 15-40. L'amide de Weinreb de formule (V) est obtenu par couplage du dérivé acide de formule (VI) ou de l'un de ses dérivés réactifs avec une N,O-dialkylamine selon les méthodes décrites dans les références ci-dessus.

Le couplage peut être réalisé en présence d'un agent de couplage tel que CDI, EDCI, HATU ou HBTU et d'une base telle que la diisopropyléthylamine, la triéthylamine ou la pyridine, dans un solvent inerte tel que le THF, le DMF ou le dichlorométhane. Alternativement on peut faire réagir la N,O-dialkylamine avec un ester de l'acide de formule (VI) en présence d'un catalyseur tel que le triméthylaluminium (Levitt. J. I.; Turos. E.; Weinreb. S. M. Synth. Commun. 1982, 12, 989.).

On peut également selon une troisième voie de synthèse (B, E) faire réagir le dérivé organométallique de formule générale (IV) défini comme ci-dessus sur un acide imidazo[1,2-*a*]pyridine-2-carboxylique de formule générale (VI) dans lequel R₁, R₂, R₃ et R₄ sont définis comme précédemment ou un de ses sels ou dérivés réactifs tels que ester, halogénure d'acide, anhydride ou amide selon des méthodes connues de l'homme du métier, telles que décrites dans J. March, Advanced Organic Chemistry (Wiley, 5th Ed. 2001) p 567 et 1213 ou dans les références citées. Dans cette troisième voie de synthèse (B, E), on peut également faire réagir sur un dérivé réactif de l'acide imidazo[1,2-*a*]pyridine-2-carboxylique de formule générale (VI) tel qu'un anhydride mixte (qui peut être généré *in situ*), un dérivé organométallique de formule générale (IV) dans laquelle X est défini comme ci-dessus et M représente un groupe acide ou ester boronique en présence d'un catalyseur au palladium tel que le tétrakistriphénylphosphine palladium.

Les produits de formule (I), et leurs précurseurs de formule (II) ou (VI), peuvent être soumis, si désiré et si nécessaire, pour obtenir des produits de formule (I) ou être transformés en d'autres produits de formule (I), à l'une ou plusieurs des réactions de transformations suivantes, dans un ordre quelconque :
a) une réaction d'estérification ou d'amidification de fonction acide,
b) une réaction d'hydrolyse de fonction ester en fonction acide,
c) une réaction de transformation de fonction hydroxyle en fonction alcoxy,
d) une réaction d'oxydation de fonction alcool en fonction aldéhyde ou cétone,
e) une réaction de transformation des fonctions aldéhyde ou cétone en fonction alcool par réduction ou action d'un organométallique tel qu'un organomagnésien,
f) une réaction d'oxydation de groupe alcènyle en fonction aldéhyde ou cétone,
g) une réaction de deshydratation de groupe hydroxyalkyle en groupe alcényle
h) une réaction d'hydrogénation totale ou partielle de groupe alcényle ou alcynyle en groupe alcényle ou alkyle
i) une réaction de couplage catalytique d'un dérivé halogèné et d'un dérivé organométallique tel que stannique ou boronique pour introduire un substituant alkyle, alcènyle, alcynyle ou aryle,
j) une réaction de réduction d'un groupe nitro en groupe amino primaire
k) une réaction de conversion d'un groupe amino primaire ou secondaire en un groupe amino secondaire ou tertiaire par amination réductrice ou alkylation
l) une réaction de protection des fonctions réactives,
m) une réaction d'élimination des groupements protecteurs que peuvent porter les fonctions réactives protégées,
n) une réaction de salification par un acide minéral ou organique ou par une base pour obtenir le sel correspondant,
o) une réaction de dédoublement des formes racémiques en énantiomères, lesdits produits de formule (I) ainsi obtenus étant le cas échéant sous toutes les formes isomères possibles racémiques, énantiomères et diastéréoisomères.

Dans le schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les exemples suivants décrivent la préparation de certains composés conformes à l'invention. Ces exemples ne sont pas limitatifs et ne font qu'illustrer la présente invention. Les numéros des exemples renvoient à ceux donnés dans les tableaux ci-après, qui illustrent les structures chimiques et les caractéristiques spectroscopiques de quelques composés selon l'invention.

### Exemple 1 : Chlorhydrate (1:1) de (6-méthylimidazo[1,2-a]pyridin-2-yl)(phényl)méthanone

### 1.1 (6-Méthylimidazo[1,2-α]pyridin-2-yl)(phényl)méthanone

A une solution de 454 mg de 3-bromo-1-phényl-propane-1,2-dione dans 1 mL de méthanol on ajoute goutte à goutte à 4°C une solution de 179 mg de 2-amino-5-méthyl pyridine dans 4 mL de méthanol. Le mélange réactionnel est agité 15 heures à 4°C puis porté au reflux pendant 2 heures. Après évaporation du solvant sous pression réduite, le résidu est repris dans du dichlorométhane et alcalinisé par une solution normale de soude. La phase aqueuse basique est extraite par du dichlorométhane et les phases organiques réunies sont lavées à l'eau puis par une solution saturée de chlorure de sodium, séchées sur sulfate de magnésium, filtrées et concentrées à sec sous pression réduite. Le résidu est chromatographié sur une cartouche de 20 g de silice en éluant par un mélange de cyclohexane et d'acétate d'éthyle 85/15 puis 65/35. Les fractions contenant le produit attendu sont réunies et concentrées à sec sous pression réduite pour donner 120 mg de (6-méthyl-imidazo[1,2-*a*]pyridin-2-yl) phényl méthanone sous la forme d'un solide beige.
Spectre de masse (IE) : m/z 236 (pic de base) : [M⁺], m/z 208 : M⁺- [CO].

### 1.2 Chlorhydrate (1:1) de (6-méthylimidazo[1,2-α]pyridin-2-yl)(phényl)méthanone

120 mg de (6-méthyl-imidazo[1,2-α]pyridin-2-yl) phényl méthanone sont repris dans 2 mL d'éthanol et la solution est traitée par 1,5 mL d'une solution d'acide chlorhydrique 4N dans le dioxanne puis par 2 mL d'éther diéthylique. Les cristaux obtenus sont filtrés et lavés par de l'éther diéthylique puis séchés. On obtient 77 mg de chlorhydrate (1:1) de (6-méthyl-imidazo[1,2-*a*]pyridin-2-yl) phényl méthanone sous la forme d'un solide beige.

### Exemple 2 : Chlorhydrate (1:1) de phényl[6-(trifluorométhyl)imidazo[1,2-α]pyridin-2-yl]méthanone

### 2.1 Bromhydrate (1:1) de phényl[6-(trifluorométhyl)imidazo[1,2-α]pyridin-2-yl]méthanone

A une solution de 0,324 g de 2-amino-5-trifluorométhylpyridine dans 4 mL de DMF refroidie à 4°C on ajoute goutte à goutte une solution de 0,65 g de 3-bromo-1-phénylpropane-1,2-dione dans 11 mL de DMF. Le mélange réactionnel est agité 16 heures à 4°C. Le précipité est filtré et lavé par de l'éther diéthylique puis séché. On obtient 0,32 g de bromhydrate (1:1) de phényl[6-(trifluorométhyl)imidazo[1,2-*a*]pyridin-2-yl]méthanone sous la forme d'un solide blanc.
Spectre RMN ¹H (DMSO-d6, δ en ppm) : :4,91 (d, J = 14,5 Hz, 1H) ; 5,14 (d, J = 14,5 Hz, 1H) ; 7,27 (m, 1H) ; 7,64 (t, J = 7,5 Hz, 2H) ; 7,76 (t, J = 7,5 Hz, 1H) ; 8,13 (d, J = 8,0 Hz, 2H) ; 8,32 (m, 1H) ; 8,48 (m, 1H) ; 8,96 (s, 1H) ; 11,3 (s, 1H) (toutes les absorptions sont larges).
Spectre de masse (IE) : m/z 290 : [M⁺], m/z 203 (pic de base) : [M⁺]-COPh
Spectre IR (KBr): 3101; 2989; 1703; 1674; 1583; 1327; 1186; 1126; 1095; 1075; 934; 836; 723 & 692 cm⁻¹

### 2.2 Phényl[6-(trifluorométhyl)imidazo[1,2-α]pyridin-2-yl]méthanone

0,32 g de bromhydrate (1:1) de phényl[6-(trifluorométhyl)imidazo[1,2-α]pyridin-2-yl]méthanone sont placés en suspension dans 10 mL d'éthanol et le milieu réactionnel est chauffé au reflux pendant 2 heures puis concentré sous pression réduite. Le résidu est repris par 10 mL de dichlorométhane et 3 mL de solution aqueuse de soude normale. La phase organique est lavée à l'eau, séchée sur sulfate de magnésium et concentrée à sec sous pression réduite pour donner 220 mg de phényl[6-(trifluorométhyl)imidazo[1,2-*a*]pyridin-2-yl]méthanone sous la forme d'un solide jaune.
Spectre de masse (IE) : m/z 290 : [M⁺] (pic de base), m/z 261 : [M-CO]⁺.

### 2.3 Chlorhydrate (1:1) de phényl[6-(trifluorométhyl)imidazo[1,2-α]pyridin-2-yl]méthanone

120 mg de phényl[6-(trifluorométhyl)imidazo[1,2-α]pyridin-2-yl]méthanone sont repris dans 2 mL de méthanol et la solution est traitée par 2 mL d'une solution d'acide chlorhydrique 4N dans le dioxanne puis par 2 mL d'éther diéthylique. La solution est agitée 16 heures à température ambiante puis concentrée sous pression réduite. Le solide obtenu est séché pour donner 85 mg de chlorhydrate (1:1) de phényl[6-(trifluorométhyl)imidazo[1,2-*a*]pyridin-2-yl]méthanone sous la forme d'un solide beige.

### Exemple 3 : Bromhydrate (1:1) de [6-(1-hydroxy-1-méthyléthyl)imidazo[1,2-α]pyridin-2-yl](phényl)méthanone

A une solution de 0,2 g de 2-(6-amino-pyridin-3-yl)-propan-2-ol dans 10 mL de diglyme, on ajoute une solution de 0,358 g de 3-bromo-1-phényl-propane-1,2-dione dans 5 mL de THF. Le mélange réactionnel est agité 15 h à 4°C puis concentré à sec sous pression réduite. Le résidu est chromatographié sur une colonne de silice en éluant par du dichlorométhane puis par un mélange de dichlorométhane et de méthanol 98/2. Les fractions contenant le produit attendu sont réunies et concentrées à sec pour donner 60 mg de bromhydrate (1:1) de [6-(1-hydroxy-1-méthyléthyl)imidazo[1,2-*a*]pyridin-2-yl](phényl)méthanone sous la forme d'un solide jaune-paille.

### Exemple 4 : [6-(Hydroxyméthyl)imidazo[1,2-α]pyridin-2-yl](phényl)méthanone

A une solution de 0,12 g de 5-hydroxyméthyl-pyridine dans 5 mL de DME, on ajoute une solution de 0,7 g de 3-bromo-1-phénylpropane-1,2-dione dans 5 mL de DME. Le mélange réactionnel est agité 15 h à 20°C puis chauffé au reflux pendant 4 heures. Le milieu réactionel est concentré à sec sous pression réduite. Le résidu est repris par 50 mL de solution saturée de bicarbonate de sodium et 50 mL d'acétate d'éthyle. La phase aqueuse est extraite 2 fois par 50 mL d'acétate d'éthyle et les phases organiques réunies sont lavées à l'eau, séchées et concentrées à sec. Le résidu est chromatographié sur une cartouche de silice en éluant avec un mélange de dichlorométhane et d'acétate d'éthyle 90/10. Les fractions contenant le produit attendu sont réunies et évaporées à sec sous pression réduite pour donner 33 mg de [6-(hydroxyméthyl)imidazo[1,2-*a*]pyridin-2-yl](phényl)méthanone sous la forme d'un solide beige.

### Exemple 5 : (6-Chloroimidazo[1,2-α]pyridin-2-yl)(2-méthylphényl)méthanone

A une solution de 0,144 g de *N*-méthoxy *N*-méthyl 6-chloroimidazo[1,2-α]pyridine-2-carboxamide dans 3 mL de THF refroidie à -4°C, on ajoute goutte à goutte une solution de 1,5 mL de solution de chlorure de 2-méthylphénylmagnésium 2M dans l'éther éthylique. Le mélange réactionnel est agité 3 heures à -4°C. On ajoute 3 mL d'acide chlorhydrique 1N, 8 mL d'eau et 40 mL d'acétate d'éthyle. Après décantation, la phase organique est séchée sur sulfate de magnésium, filtrée, évaporée à sec sous pression réduite, puis purifiée sur une colonne de silice en éluant par un mélange de dichlorométhane et d'acétate d'éthyle 90/10 en volume. Les fractions contenant le produit sont réunies et concentrées à sec sous pression réduite pour donner 0,034 g de (6-chloroimidazo[1,2-*a*]pyridin-2-yl)(2-méthylphényl)méthanone sous la forme d'un solide blanc.

### Exemple 6 : Phényl(6-phénylimidazo[1,2-α]pyridin-2-yl)méthanone

Dans un tube à microondes de 20 mL on charge 0,391 g de (6-bromoimidazo[1,2-α]pyridin-2-yl)(phényl)méthanone, 0,237 g d'acide phénylboronique, 45 mg de tétrakis(triphénylphosphine)-palladium, 4 mL de solution aqueuse 2M de carbonate de sodium, 6 mL d'acétonitrile et 6 mL de toluène. Le mélange est agité 20 minutes dans un appareil à microondes réglé sur 150°C. Après refroidissement, la phase organique est séparée, séchée et évaporée. Le résidu est repris par un mélange de dichlorométhane et de pentane. Le solide est filtré et puis purifié par trituration dans du méthanol pour donner 0,16 g de phényl(6-phénylimidazo[1,2-α]pyridin-2-yl)méthanone sous la forme d'un solide écru.

### Exemple 7 : [5-(2-méthoxyéthoxy)imidazo[1,2-α]pyridin-2-yl](phényl)méthanone

A 800 µL de solution 2,5 M de n-butyllithium dans l'hexane, on ajoute 1,2 mL de 2 méthoxyéthanol sec puis laisse revenir à 20°C. On ajoute 150 mg de (5-bromoimidazo[1,2-a]pyridin-2-yl)(phényl)méthanone et chauffe 20 minutes à 120° dans un appareil à microondes. Le mélange réactionnel refroidi est repris par 50 mL d'eau et 20 mL d'acétate d'éthyle. La phase aqueuse est réextraite 2 fois par 20 mL d'acétate d'éthyle et les phases organiques réunies sont lavées par 50 mL de saumure, séchées sur sulfate de magnésium et concentrées. Le résidu est chromatographié sur une colonne de silice en éluant par un mélange de dichlorométhane et de méthanol 95/5. Les fractions contenant le produit attendu sont réunies et concentrées à sec sous pression réduite pour donner, après concrétion à l'éther éthylique, 36 mg de [5-(2-méthoxyéthoxy)imidazo[1,2-*a*]pyridin-2-yl](phényl)méthanone sous la forme d'un solide rosé.

### Exemple 8 : N-(2-benzoylimidazo[1,2-α]pyridin-6-yl)acétamide

### 8.1 (6-Nitroimidazo[1,2-α]pyridin-2-yl)(phényl)méthanone

A une solution de 0,48 g de 3-bromo-1-phénylpropane-1,2-dione dans un mélange de 3 mL de THF et 0,5 mL d'éthanol on ajoute 0,25 g de 2-amino-5-nitropyridine. Le mélange réactionnel est chauffé 15 minutes à 160° dans un appareil à microondes. Le solide formé est séparé, repris dans 3 mL d'éthanol et chauffé au reflux pendant 6 heures. Après évaporation du solvant le résidu est repris par de l'acétate d'éthyle et une solution de soude diluée. La phase organique est lavée à l'eau et séchée puis évaporée pour donner 0,42 g de (6-nitroimidazo[1,2-α]pyridin-2-yl)(phényl)méthanone sous la forme d'un solide marron, utilisé sans autre purification.
Spectre RMN ¹H (DMSO-d6, δ en ppm) : 7,60 (t large, J = 7,5 Hz, 2H) ; 7,71 (t large, J = 7,5 Hz, 1H) ; 7,88 (d large, J = 10,0 Hz, 1H) ; 8,06 (dd, J = 2,5 et 10,0 Hz, 1H) ; 8,30 (d large, J = 8,0 Hz, 2H) ; 8,84 (d, J = 1,0 Hz, 1H) ; 9,94 (dd, J = 1,0 et 2,5 Hz, 1H).
Spectre de masse (IC) : m/z 268 (pic de base), [M+H]⁺, m/z 285 : [M+NH₄]⁺.

### 8.2 (6-Aminoimidazo[1,2-α]pyridin-2-yl)(phényl)méthanone

0,42 g de 2-benzoyl-6-nitro-imidazo[1,2-α]pyridine dans 20 mL d'éthanol bouillant sont traités par 10 mL de solution 0,5 N de soude et 2,77 g de dithionite de sodium. Après 10 minutes à 80°C le milieu réactionnel est refroidi, filtré et concentré à sec pour donner la (6-aminoimidazo[1,2-*a*]pyridin-2-yl)(phényl)méthanone brute utilisée sans autre purification.

### 8.3 N-(2-benzoylimid azo[1,2-α] pyridin-6-yl)acétamide

La (6-aminoimidazo[1,2-α]pyridin-2-yl)(phényl)méthanone est dissoute dans 7 mL d'acide acétique et traitée par 0,8 mL d'anhydride acétique. Le milieu réactionnel est chauffé au reflux pendant 2 heures, refroidi, filtré et concentré à sec. Le résidu est repris par de l'acétate d'éthyle et une solution saturée d'hydrogènocarbonate de sodium. La phase organique est lavée et concentrée et le produit obtenu est chromatographié sur une colonne de silice en éluant par un mélange de dichlorométhane et de méthanol 95/5. Les fractions contenant le produit attendu sont réunies et concentrées pour donner 150 mg de *N*-(2-benzoylimidazo[1,2-*a*]pyridin-6-yl)acétamide en mélange avec la *N*-(3-benzoylimidazo[1,2-α]pyridin-6-yl)acétamide. Les deux isomères sont séparés par LC/MS préparative de façon à donner 54 mg de *N*-(2-benzoyl-imidazo[1,2-*a*]pyridin-6-yl)-acétamide purifiée sous la forme d'un solide beige.

### Exemple 9 : (6-Isopropénylimidazo[1,2-α]pyridin-2-yl)(phényl)méthanone

300 mg de bromhydrate (1:1) de [6-(1-hydroxy-1-méthyléthyl)imidazo[1,2-α]pyridin-2-yl](phényl)méthanone sont repris dans un mélange de 100 mL de dichlorométhane et 30 mL de solution saturée de bicarbonate de sodium. La phase organique est décantée, séchée et concentrée à sec pour donner 190 mg de [6-(1-hydroxy-1-méthyléthyl)imidazo[1,2-α]pyridin-2-yl](phényl)méthanone que l'on dissout dans 5 mL de xylène et porte au reflux pendant 2 heures après addition de 6,5 mg d'acide paratoluènesulfonique. Après 16 heures à 20°C et évaporation du solvant le résidu est chromatographié sur une cartouche de 6 g de silice en éluant par un mélange de dichlorométhane et de méthanol 95/5. Les fractions contenant le produit attendu sont évaporées à sec et le résidu trituré dans de l'éther éthylique pour donner 60 mg de (6-isopropénylimidazo[1,2-*a*]pyridin-2-yl)(phényl)méthanone sous la forme d'un solide jaune pâle.

### Exemple 10 : 2-Benzoyl-N,N-diméthylimidazo[1,2-α]pyridine-6-carboxamide

### 10.1 Bromhydrate (1:1) de l'acide 2-benzoylimidazo[1,2-α]pyridine-6-carboxylique

A une suspension de 0,33 g d'acide 6-aminonicotinique dans 5 mL de THF et 3 mL d'éthanol on ajoute une solution de 0,654 g de 3-bromo-1-phényl-propane-1,2-dione dans 2 mL de THF. Le mélange réactionnel est agité à 45° pendant 16 heures et 24 heures à 20°C puis évaporé à sec. Le résidu est repris par du dichlorométhane et le solide est filtré et lavé au dichlorométhane pour donner 0,78 g de bromhydrate de l'acide 2-benzoylimidazo[1,2-α]pyridine-6-carboxylique sous la forme d'un solide jaune.

### 10.2 2-Benzoyl-N,N-diméthylimidazo[1,2-α]pyridine-6-carboxamide

Une solution de 0,21 g de bromhydrate de l'acide 2-benzoylimidazo[1,2-*a*]pyridine-6-carboxylique, 0,35 g de chlorhydrate d'EDCI et 0,294 g d'HOBT dans 7 mL de DMF est agitée 30 minutes à 20°C puis on ajoute 1 mL de solution 1 M de diméthylamine dans le THF et agite 2 heures à 20°C. Le mélange réactionnel est concentré à sec puis repris par 100 mL d'eau et 200 mL d'acétate d'éthyle. La phase organique est séparée, séchée et évaporée. Le résidu est purifié par flash-chromatographie sur une colonne de 20 g de silice en éluant par un mélange de dichlorométhane et de méthanol 99/1. Les fractions contenant le produit attendu sont réunies et concentrées sous vide pour donner 40 mg de 2-benzoyl-*N,N*-diméthylimidazo[1,2-α]pyridine-6-carboxamide sous la forme d'un solide beige.

### Exemple 11 : 2-Benzoyl-N-méthylimidazo[1,2-α]pyridine-6-carboxamide

En opérant comme dans l'exemple 10, en remplaçant la diméthylamine par la méthylamine, on obtient 29 mg de 2-benzoyl-*N*-méthylimidazo[1,2-α]pyridine-6-carboxamide sous la forme d'une poudre écrue.

### Exemple 12 : Phényl(6-vinylimidazo[1,2-α]pyridin-2-yl)méthanone

Un mélange de 0,4 g de (6-iodolimidazo[1,2-α]pyridin-2-yl)(phényl)méthanone, 120 mg de tétrakis(triphénylphosphine)palladium(0), 336 µL de tributylvinylétain et 20 mL de DMF est chauffé 5 minutes à 130°C dans un appareil à microondes puis de nouveau 5 minutes à 130°C après ajout de 70 mg de tétrakis(triphénylphosphine)palladium(0) et 200 µL de tributylvinylétain. Le mélange réactionnel est concentré à sec, repris dans 50 mL d'eau et extrait par deux fois 50 mL d'acétate d'éthyle. Les phases organiques réunies sont lavées par une solution saturée de chlorure de sodium, séchées sur sulfate de magnésium et évaporées à sec sous pression réduite. Le résidu est chromatographié sur une cartouche de silice en éluant par un mélange de cyclohexane et d'acétate d'éthyle (gradient de 10 à 30 %). Les fractions contenant le produit attendu sont réunies et concentrées à sec sous pression réduite pour donner 0,13 g de phényl(6-vinylimidazo[1,2-*a*]pyridin-2-yl)méthanone sous la forme d'un solide jaune.

### Exemple 13 : (6-Ethylimidazo[1,2-α]pyridin-2-yl)(phényl)méthanone

Une solution de 80 mg de phényl(6-vinylimidazo[1,2-α]pyridin-2-yl)méthanone dans 20 mL de méthanol, est hydrogénée pendant 45 minutes à 45°C sous 1 bar d'hydrogène en présence de 34 mg de palladium à 10 % sur charbon. Le produit est chromatographié sur silice en éluant par un gradient de cyclohexane et d'acétate d'éthyle pour donner 15 mg de (6-éthylimidazo[1,2-*a*]pyridin-2-yl)(phényl)méthanone sous la forme d'un solide blanc.

Les intermédiaires décrits ci-dessous sont utiles à la préparation des composés de la présente invention.

### 2-(6-Amino-pyridin-3-yl)-propan-2-ol

A une solution de 0,7 g de 6-amino-nicotinate de méthyle dans 65 mL de THF, refroidie à 10°C et sous argon, on ajoute goutte à goutte 15 mL de solution 3 M de chlorure de méthylmagnésium dans le THF. Le mélange réactionnel est agité 15 h en laissant remonter la température à 20°C, puis refroidi de nouveau dans un bain de glace. On ajoute lentement 100 mL de solution saturée de chlorure d'ammonium puis 200 mL d'acétate d'éthyle. La phase organique est séchée et concentrée à sec. Le résidu est repris dans de l'acétate d'éthyle. Le précipité est essoré et séché pour donner 0,4 g de 2-(6-Amino-pyridin-3-yl)-propan-2-ol sous la forme d'un solide jaune pâle.
Spectre RMN 1H (DMSO-d6, δ en ppm) : 1,36 (s, 6H) ; 4,82 (s, 1H) ; 5,67 (s large, 2H) ; 6,37 (d, J = 9,0 Hz, 1H) ; 7,42 (dd, J = 2,5 et 9,0 Hz, 1H) ; 7,98 (d, J = 2,5 Hz, 1H) Spectre de masse (IE) : m/z 152 : [M+.], m/z 137 : [M+.]-CH3 (pic de base)

### 1-[6-(2,5-Diméthylpyrrol-1-yl)-pyridin-3-yl]-propan-1-ol

A une solution de 2 g de 5-bromo-2-(2,5-diméthylpyrrol-1-yl)-pyridine dans 30 mL de tert-butyl-méthyléther refroidie à -78°C on ajoute lentement sous argon 3,3 mL d'une solution 2,5 M de butyllithium dans l'hexane puis agite 40 minutes vers -70°C avant d'ajouter une solution de 0,5 g de propionaldéhyde dans 5 mL de tert-butyl-méthyléther. On agite 30 minutes vers -70°C puis ajoute lentement 20 mL d'eau et laissse revenir à 20°C. La phase aqueuse est extraite par 2 fois 100 mL d'acétate d'éthyle et les phases organiques sont réunies, séchées et concentrées à sec. Le résidu est purifié par flash-chromatographie sur une cartouche de 70 g de silice en éluant par du dichlorométhane puis par un mélange de dichlorométhane et d'acétate d'éthyle 80/20. Les fractions contenant le produit attendu sont réunies et concentrées sous vide pour donner 1,07 g de 1-[6-(2,5-diméthylpyrrol-1-yl)-pyridin-3-yl]-propan-1-ol sous la forme d'une huile jaune.
Spectre RMN 1H (DMSO-d6, δ en ppm) : 0,87 (t, J = 7,5 Hz, 3H) ; 1,69 (m, 2H) ; 2,02 (s, 6H) ; 4,60 (m, 1H) ; 5,37 (d, J = 4,5Hz, 1H) ; 5,78 (s, 2H) ; 7,34 (d, J = 8,0 Hz, 1H) ; 7,89 (dd, J = 2,5 et 8,0 Hz, 1H) ; 8,51 (d, J = 2,5 Hz, 1H).
Spectre de masse (IE) : m/z=230 [M]+ (pic de base) m/z=215 [M - CH3]+.

### 2-amino-5-(1-éthoxypropyl)pyridine

A une solution de 1,07 g de 1-[6-(2,5-diméthylpyrrol-1-yl)-pyridin-3-yl]-propan-1-ol dans 20 mL d'éthanol chauffée à 80°C, on ajoute une solution de 1,94 g de chlorhydrate d'hydroxylamine dans 1,6 mL d'eau et agite 24 heures à 80°C. Après refroidissement à température ambiante le milieu réactionnel est filtré et concentré à sec. Le résidu est purifié par flash-chromatographie sur une colonne de 100 g de silice en éluant par 400 mL de dichlorométhane puis 300 mL d'acétate d'éthyle puis par un mélange de dichlorométhane et de méthanol 95/5. Les fractions contenant le produit attendu sont réunies et concentrées sous vide pour donner 0,186 g de 2-amino-5-(1-éthoxypropyl)pyridine sous la forme d'une huile brune.
Spectre RMN 1H (DMSO-d6, δ en ppm) : 0,78 (t, J = 7,5 Hz, 3H) ; 1,06 (t, J = 7,0 Hz, 3H) ; 1,53 (m, 1H) ; 1,70 (m, 1H) ; 3,24 (q partiellement masqué, J = 7,0 Hz, 2H) ; 4,02 (t, J = 7,0 Hz, 1H) ; 5,48 (m large, 2H) ; 6,61 (d, J = 9,0 Hz, 1H) ; 7,47 (dd, J = 2,5 et 9,0 Hz, 1H) ; 7,79 (d, J = 2,5 Hz, 1H).
Spectre de masse (IE) : m/z=180 [M]+, m/z=151 [M - C2H5]+, m/z=123 [m/z=151 - C2H4]+, m/z=77 [C6H5]+ (pic de base).

### 6-Aminopyridine-3-carboxaldéhyde

A une suspension de 1 g de 6-aminopyridine-3-carbonitrile dans 20 mL de toluène refroidie à -78°C on ajoute goutte à goutte 15 mL d'une solution 1,2 M d'hydrure de diisobutylaluminium dans le toluène. Le milieu réactionnel est agité 30 minutes à -78°C puis en laissant lentement remonter à 20°C. Après avoir refroidi à nouveau à -78°C on ajoute lentement 6 mL d'eau et agite pendant 1 heure à -78°C. Le milieu réactionnel est agité 16 heures à 20°C, traité par 20 mL d'acide chlorhydrique 2,5 N, agité 20 minutes, alcalinisé par addition de soude concentrée et extrait 3 fois par 100 mL de dichlorométhane. Les phases organiques réunies sont séchées et concentrées à sec sous pression réduite. Le résidu est repris par 40 mL d'acétate d'éthyle et 40 mL de solution saturée de bisulfite de sodium. La phase aqueuse est lavée 2 fois par 40 mL d'acétate d'éthyle, alcalinisée vers pH 14 par addition de soude concentrée et extraite 3 fois par 50 mL de dichlorométhane. Les phases organiques réunies sont séchées et concentrées à sec sous pression réduite pour donner 200 mg 6-aminopyridine-3-carboxaldéhyde brut sous la forme d'une poudre jaune qui est utilisée sans autre purification.

### Acide 6-bromo-5-méthylimidazo[1,2-α]pyridine-2-carboxylique

A une solution de 0,283 g de 6-bromo-5-méthylimidazo[1,2-α]pyridine-2-carboxylate d'éthyle dans 2 mL d'éthanol, on ajoute 1 mL d'hydroxyde de sodium 1 N. Le mélange réactionnel est chauffé à reflux pendant 3 heures. Après refroidissement, le milieu réactionnel est acidifié avec l'acide acétique. Le solide formé est filtré, lavé à l'eau puis séché sous vide pour donner 0,195 g de l'acide 6-bromo-5-méthylimidazo[1,2-*a*]pyridine-2-carboxylique sous la forme d'un solide blanc.
Spectre RMN 1H (DMSO-d6, δ en ppm) : 2,77 (s, 3H) ; de 7,46 à 7,53 (m, 2H) ; 8,42 (s, 1H).
Spectre de masse (IE) : m/z 254 (pic de base) : [M+.], m/z 210 : [M+.]-CO2H, m/z 170 : 210-C2HN

### N-méthoxy-N-méthyl-6-chloroimidazo[1,2-α]pyridine-2-carboxamide

A une solution de 0,784 g de l'acide 6-chloroimidazo[1,2-α]pyridine-2-carboxylique dans 12 mL de dichlorométhane, on ajoute 1,67 mL de triéthylamine, 1,53 g de chlorhydrate de 1-(3-diméthylaminopropyl)-3-éthylcarbodiimide et 1,08 g de 1-hydroxybenzotriazole. Le mélange réactionnel est agité 20 minutes à la température ambiante. On ajoute 0,39 g de chlorhydrate de N-O-diméthyl-hydroxylamine. Le mélange réactionnel est agité 4 heures à température ambiante. On ajoute 60 mL de dichlorométhane et 30 mL d'eau. Après décantation, la phase organique est séchée sur sulfate de magnésium, filtrée, évaporée à sec sous pression réduite, puis purifiée sur une colonne de silice en éluant par un mélange de dichlorométhane et de méthanol 95/05 en volume. Les fractions contenant le produit sont réunies et concentrées à sec sous pression réduite pour donner 0,6 g de *N*-méthoxy *N*-méthyl 6-chloroimidazo[1,2-*a*]pyridine-2-carboxamide sous la forme d'un solide blanc.
Spectre RMN 1H (DMSO-d6, δ en ppm) : 3,42 (s large, 3H) ; 3,75 (s, 3H) ; 7,37 (dd, J = 2,0 et 9,5 Hz, 1H) ; 7,67 (d, J = 9,5 Hz, 1H) ; 8,39 (s, 1H) ; 8,85 (d, J = 2,0 Hz, 1H).
Spectre de masse (LCMS) : m/z 240 : [M+H]⁺.

### N-méthoxy-N-méthyl-5-méthylimidazo[1,2-α]pyridine-2-carboxamide

Le *N*-métlioxy-*N*-méthyl-5-méthylimidazo[1,2-α]pyridine-2-carboxamide est préparé selon le mode opératoire décrit ci-dessus à partir de l'acide 5-méthylimidazo[1,2-*a*]pyridine-2-carboxylique.
Spectre RMN 1H (DMSO-d6, δ en ppm) : 2,64 (s, 3H) ; 3,47 (s large, 3H) ; 3,77 (s, 3H) ; 6,85 (d large, J = 7,0 Hz, 1H) ; 7,30 (dd, J = 7,0 et 9,0 Hz, 1H) ; 7,51 (d large, J = 9,0 Hz, 1H) ; 8,21 (s, 1H).
Spectre de masse (IE) : m/z 219 : [M+.], m/z 188 : [M+.]-OCH3 , m/z 159 (pic de base) : [M+.]-C2H6NO.

### N-méthoxy-N-méthyl-6-bromo-5-méthylimidazo[1,2-α]pyridine-2-carboxamide

Le *N*-méthoxy-*N*-méthyl-6-bromo-5-méthylimidazo[1,2-α]pyridine-2-carboxamide est préparé selon le mode opératoire décrit ci-dessus à partir de l'acide 6-bromo-5-méthylimidazo[1,2-*a*]pyridine-2-carboxylique.
Spectre RMN 1H (DMSO-d6, δ en ppm) : 2,77 (s, 3H) ; 3,45 (s large, 3H) ; 3,77 (s, 3H) ; de 7,48 à 7,53 (m, 2H) ; 8,33 (s, 1H).
Spectre de masse (IE) : m/z 297 : [M⁺], m/z 266 : [M⁺]-OMe , m/z 237 (pic de base) : [M⁺]-C2H6NO.

### N,N-diméthyl-6-nitro-pyridine-3-amine

A une solution de 1 g de 5-bromo-2-nitropyridine dans 5 mL d'éthanol on ajoute 6 mL de solution 2 M de diméthylamine dans le tétrahydrofuranne. Le mélange réactionnel est chauffé 2 heures à 140°C dans un appareil à microondes. Après refroidissement, le solide formé est séparé et lavé à l'éther éthylique pour donner 850 mg de *N,N*-diméthyl-6-nitro-pyridine-3-amine sous la forme d'un solide jaune.
Spectre RMN 1H (DMSO-d6, δ en ppm) : 3,12 (s, 6H) ; 7,21 (dd, J = 3,0 et 9,5 Hz, 1H) ; 8,02 (d, J = 3,0 Hz, 1H) ; 8,15 (d, J = 9,5 Hz, 1H)
Spectre de masse (IE) : m/z 167 (pic de base) : [M⁺], m/z 137 : [M⁺]- NO , m/z 121: [M+.]- NO₂.

### 5-Diméthylaminopyridine-2-amine

La *N*,*N*-diméthyl-6-nitro-pyridine-3-amine obtenue ci-dessus est reprisse dans 25 mL d'éthanol. Après ajout de 4,8 g de chlorure stanneux, le mélange réactionnel est chauffé au reflux pendant 30 minutes puis concentré à sec. Le résidu est chromatographié sur une colonne de silice en éluant par un mélange de dichlorométhane et de méthanol ammoniacal 90/10. Les fractions contenant le produit attendu sont réunies et concentrées pour donner 750 mg de 5-diméthylaminopyridine-2-amine sous la forme d'un solide pâteux jaune.
Spectre RMN 1H (DMSO-d6, δ en ppm) : 2,80 (s, 6H) ; 6,94 (d, J = 9,5 Hz, 1H) ; 7,18 (d, J = 3,0 Hz, 1H) ; 7,32 (s large, 2H) ; 7,83 (dd, J = 3,0 et 9,5 Hz, 1H)
Spectre de masse (IE) : m/z 137 (pic de base) : [M+.], m/z 122 : [M+.]-CH3.

### 6-Diméthylaminoimidazo[1,2-α]pyridine-2-carboxylate d'éthyle

A une suspension de 0,2 g de 5-diméthylaminopyridine-2-amine dans 3 mL de DME on ajoute 215 µL de bromopyruvate d'éthyle. Le mélange réactionnel est agité à 20°C pendant 16 heures puis, après ajout de 3 mL d'éthanol, pendant 16 heures au reflux et enfin concentré sous pression réduite. Le résidu est filtré sur une cartouche de 15 g de silice en éluant par un mélange de dichlorométhane et de méthanol (98/2). Les fractions contenant le produit attendu sont réunies et lavées par par une solution saturée de bicarbonate de sodium. La phase organique est séchée et concentrée à sec sous pression réduite pour donner 76 mg de 6-diméthylaminoimidazo[1,2-*a*]pyridine-2-carboxylate d'éthyle sous la forme d'une huile verte utilisée telle quelle dans la suite de la synthèse.

Les tableaux qui suivent illustrent les structures chimiques (tableau 1) et les caractéristiques spectroscopiques (tableau 2) de quelques exemples de composés selon l'invention.

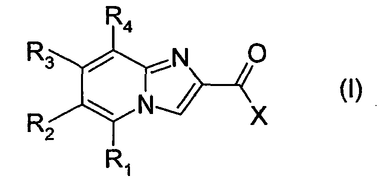

**Tableau 1**

| **Composé** | **R₁** | **R₂** | **R₃** | **R₄** | **X** | **Sel** |
|---|---|---|---|---|---|---|
| **1** | H | Me | H | H | Ph | HCl |
| **2** | H | ~CF₃ | H | H | Ph | HCl |
| **3** | H | ~CMe₂OH | H | H | Ph | HBr |
| **4** | H | ~CH₂OH | H | H | Ph | |
| **5** | H | Cl | H | H | 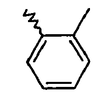 | |
| **6** | H | Ph | H | H | Ph | |
| **7** | 2-Méthoxy- éthoxy | H | H | H | Ph | |
| **8** | H | ~NHCOMe | H | H | Ph | |
| **9** | H | ~CMe=CH₂ | H | H | Ph | |
| **10** | H | ~CONMe₂ | H | H | Ph | |
| **11** | H | ~CONHMe | H | H | Ph | |
| **12** | H | ~CH=CH₂ | H | H | Ph | |
| **13** | H | ~CH₂CH₃ | H | H | Ph | |
| **14** | H | F | H | H | Ph | |
| **15** | H | Cl | H | Cl | Ph | HCl |
| **16** | H | H | Cl | H | Ph | |
| **17** | H | Br | H | H | Ph | |
| **18** | Br | H | H | H | Ph | |
| **19** | H | ~CN | H | H | Ph | |
| **20** | NH₂ | H | H | H | Ph | |
| **21** | H | ~CONH₂ | H | H | Ph | |
| **22** | H | I | H | H | Ph | HBr |
| **23** | H | H | OH | H | Ph | HBr |
| **24** | H | F | H | F | Ph | HBr |
| **25** | H | ~CO₂Me | H | H | Ph | HBr |
| **26** | H | 1-éthoxy-propyl H | | H | Ph | |
| **27** | H | ~COMe | H | H | Ph | |
| **28** | H | ~CH=O | H | H | Ph | |
| **29** | H | Cl | H | H | 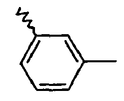 | |
| **30** | H | Cl | H | H | 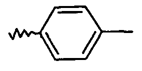 | |
| **31** | H | Cl | H | H | 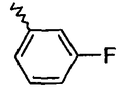 | |
| **32** | H | Cl | H | H | 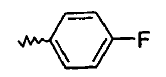 | |
| **33** | H | Cl | H | H | 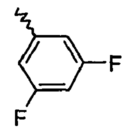 | |
| **34** | Me | H | H | H | Ph | |
| **35** | Me | Br | H | H | Ph | |
| **36** | H | Cl | H | H | 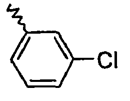 | |
| **37** | H | Cl | H | H | 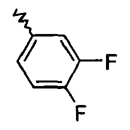 | |
| **38** | H | ~N(CH₃)₂ | H | H | Ph | PF₆ |
| **39** | H | 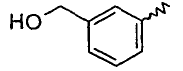 | H | H | Ph | |
| **40** | H | 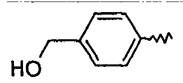 | H | H | Ph | |
| **41** | H | 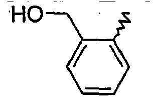 | H | H | Ph | |
| **42** | H | 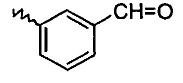 | H | H | Ph | |
| **43** | Me | Me | H | H | Ph | |

**Tableau 2**

| **Composé** | **Caractérisations** |
|---|---|
| **1** | Spectre RMN ¹H (DMSO-d6, δ en ppm) : 2,40 (s, 3H ; 7,65 (m, 3H) ; 7,77 (t large, J = 7,5 Hz, 2H) ; 8,18 (d large, J = 8,0 Hz, 2H) ; 8,57 (s large, 1H) ; 8,79 (s, 1H). Spectre de masse (IE) : m/z 236 : [M^{+.}] (pic de base), m/z 208 : [M-CO]⁺. Spectre IR (KBr) : 3047; 2796; 1650; 1565; 1259; 1229; 917; 806; 727 & 702 cm⁻¹ |
| **2** | Spectre RMN ¹H (DMSO-d6, δ en ppm) : de 7,55 à 7,76 (m, 4H) ; 7,94 (d, J = 8,5 Hz, 1H) ; 8,30 (d large, J = 8,0 Hz, 2H) ; 8,74 (s, 1H) ; 9,32 (s large, 1H). Spectre de masse (IE) : m/z 290 : [M⁺.] (pic de base), m/z 261 : [M-CO]⁺. Spectre IR (KBr) : 3062; 2748; 1665; 1577; 1385; 1340; 1297; 1223; 1170; 1140; 1066; 916; 723 & 673 cm⁻¹. |
| **3** | Spectre RMN ¹H (DMSO-d6, δ en ppm) : 1,50 (s, 6H) ; 7,60 (t large, J = 7,5 Hz, 2H) ; de 7,67 à 7,74 (m, 3H) ; 8,23 (d large, J = 8,0 Hz, 2H) ; 8,71 (s large, 1H) ; 8,77 (s large, 1H). Spectre de masse (IE) : m/z 280 (pic de base) : [M]⁺, m/z 265 : [M-CH₃]⁺, m/z 105 : [PhCO]⁺ Spectre IR (KBr) : 2975 ; 1657 ; 1596 ; 1558 ; 1286 ; 1180 ; 914 & 723 cm⁻¹. |
| **4** | Spectre RMN¹H (DMSO-d6, δ en ppm) : 4,54 (dd, J = 1,5 et 6,0 Hz, 2H) ; 5,44 (t, J = 6,0 Hz, 1H) ; 7,32 (dd, J = 1,5 et 9,5 Hz, 1H) ; 7,57 (t large, J = 7,5 Hz, 2H) ; de 7,64 à 7,70 (m, 2H) ; 8,32 (d large, J = 8,0 Hz, 2H) ; 8,53 (m large, 1H) ; 8,65 (d, J = 1,0 Hz, 1H). Spectre IR (KBr) : 3393; 1624; 1599; 1546; 1275; 1257; 1240; 1061; 899; 802; 725 & 694 cm⁻¹. Spectre de masse (IE) : m/z=252 [M]⁺ (pic de base), m/z=223 [M - CHO]⁺, m/z=105 [PhCO]⁺, m/z=77 [C₆H₅]⁺. |
| **5** | Spectre RMN¹H (DMSO-d6, δ en ppm) : 2,31 (s, 3H) ; de 7,29 à 7,39 (m, 2H) ; de 7,41 à 7,50 (m, 2H) ; 7,60 (d large, J = 8,0 Hz, 1H) ; 7,71 (d, J = 10,0 Hz, 1H) ; 8,44 (s, 1H) ; 8,85 (d, J = 2,5 Hz, 1H): Spectre de masse (IE) : m/z 270 : [M^{+.}], m/z 241 (pic de base) : [M-CO]⁺. |
| **6** | Spectre RMN ¹H (DMSO-d6, δ en ppm) : 7,44 (t large, J = 7,5 Hz, 1H) ; 7,54 (t large, J = 7,5 Hz, 2H) ; 7,59 (t large, J = 7,5 Hz, 2H) ; 7,69 (t large, J = 7,5 Hz, 1H) ; de 7,72 à 7,79 (m, 3H) ; 7,82 (d, J = 9,5 Hz, 1H) ; 8,34 (d large, J = 8,0 Hz, 2H) ; 8,64 (s, 1H) ; 9,00 (s large, 1H). Spectre IR (KBr) : 1643; 1542; 1503; 1264; 893; 758; 716; 698 & 684 cm⁻¹. Spectre de masse (ES) : m/z=299 [MH]⁺ (pic de base). |
| **7** | Spectre RMN ¹H (DMSO-d6, δ en ppm) : 3,37 (s, 3H) ; 3,82 (m, 2H) ; 4,50 (m, 2H) ; 6,53 (d, J = 7,5 Hz, 1H) ; 7,35 (d, J = 7,5 Hz, 1H) ; 7,44 (t, J = 7,5 Hz, 1H) ; 7,58 (t, J = 7,5 Hz, 2H) ; 7,68 (t, J = 7,5 Hz, 1H) ; 8,29 (s, 1H) ; 8,31 (d, J = 8,0 Hz, 2H) (absorptions larges): Spectre de masse (IE) : m/z 296 (pic de base) : [M^{+.}], m/z 238 : [M^{+.}]-CH₃O(CH₂)₂, m/z 59 : CH₃O(CH₂)₂⁺. Spectre IR (KBr) : 3182; 1643; 1543; 1344; 1271; 1237; 1126; 1024; 914; 853; 772; 731; 704 & 679 cm⁻ |
| **8** | Spectre RMN¹H (DMSO-d6, δ en ppm) : 2,11 (s, 3H) ; 7,28 (dd, J = 2,0 et 9,5 Hz, 1H) ; 7,56 (t large, J = 7,5 Hz, 2H) ; 7,66 (m, 2H) ; 8,30 (d large, J = 8,0 Hz, 2H) ; 8,73 (s 1H) ; 9,32 (s large, 1H) ; 10,2 (s large, 1H). Spectre de masse (IE) : m/z 279 : [M^{+.}], m/z 237 : [M^{+.}]-COCH3, m/z 43 (pic de base) : COCH₃⁺. Spectre IR (KBr) : 3267; 1668; 1638; 1539; 1369; 1280; 1243; 1012; 894; 813 & 718 cm⁻¹. |
| **9** | Spectre RMN ¹H (DMSO-d6, δ en ppm) : 2,15 (s, 3H) ; 5,25 (s large, 1H) ; 5,63 (s, 1H) ; 7,58 (t large, J = 7,5 Hz, 2H) ; de 7,64 à 7,73 (m, 3H) ; 8,32 (d large, J = 8,0 Hz, 2H) ; 8,60 (s, 1H) ; 8,73 (s large, 1H). Spectre IR (KBr) : 1636; 1577; 1539; 1268; 1230; 897; 808 & 708 cm⁻¹ Spectre de masse (IE) m/z=262 [M]⁺, m/z=233 [M - C₂H₅]⁺, m/z=105 [C₇H₅O]⁺, m/z=77 [C₆H₅]⁺ (pic de base). |
| **10** | Spectre RMN¹H (DMSO-d6, δ en ppm) : 3,03 (s, 6H) ; 7,42 (d, J = 7,5 Hz, 1H) ; de 7,51 à 7,79 (m, 4H) ; 8,30 (d, J = 8,0 Hz, 2H) ; 8,64 (s, 1H) ; 8,83 (s, 1H). Spectre IR (KBr) : 1647; 1617; 1496; 1398; 1281; 1241; 895 & 725 cm⁻¹. Spectre de masse (ES) : m/z=294 [MH]⁺ (pic de base). |
| **11** | Spectre RMN ¹H (DMSO-d6, δ en ppm) 2,81 (d, J = 4,5 Hz, 3H) ; de 7,52 à 7,73 (m, 3H) ; 7,78 (m, 2H) ; 8,31 (d, J = 8,0 Hz, 2H) ; 8,64 (m, 1H) ; 8,74 (s, 1H) ; 9,16 (s, 1H). Spectre IR (KBr) : 1650; 1626; 1599; 1576; 1554; 1532; 1271; 1235 & 719 cm⁻¹. Spectre de masse (IE) : m/z=279 [M]⁺ (pic de base), m/z=249 [M - CH₄N]⁺, m/z=105 [C₇H₅O]⁺, m/z=77 [C₆H₅]⁺. |
| **12** | Spectre RMN¹H (DMSO-d6, δ en ppm) : 5,40 (d, J=11,0 Hz, 1H) ; 5,93 (d, J = 17,5 Hz, 1H) ; 6,77 (dd, J = 11,0 et 17,5 Hz, 1H) ; 7,58 (t large, J = 7,5 Hz, 2H) ; 7,67 (t large partiellement masqué, J = 7,5 Hz, 1H) ; 7,69 (s large, 2H) ; 8,31 (d large, J = 8,0 Hz, 2H) ; 8,59 (s, 1H) ; 8,64 (s large, 1H). Spectre de masse (ES) : m/z 249 [M+H⁺]. |
| **13** | Spectre RMN ¹H (DMSO-d6, δ en ppm) : 1,23 (t, J = 7,5 Hz, 3H) ; 2,64 (q, J = 7,5 Hz, 2H) ; 7,31 (dd, J = 2,0 et 9,5 Hz, 1H) ; 7,57 (t, J = 7,5 Hz, 2H) ; de 7,62 à 7,70 (m, 2H) ; 8,31 (d large, J = 7,5 Hz, 2H) ; 8,43 (s large, 1H) ; 8,55 (s, 1H). |
| **14** | Spectre RMN¹H (DMSO-d6, δ en ppm) : 7,50 (ddd, J = 2,5 - 8,5 et 11,0 Hz, 1H) ; 7,59 (t large, J = 7,5 Hz, 2H) ; 7,69 (t large, J = 7,5 Hz, 1H ; 7,81 (dd, J = 5,0 et 8,5 Hz, 1H) ; 8,31 (d large, J = 8,0 Hz, 2H) ; 8,61 (s, 1H) ; 8,82 (dd, J = 2,5 et 5,0 Hz, 1H). Spectre de masse (IE) : m/z 240 : [M^{+.}] (pic de base). Spectre IR : 3146; 3059; 1644; 1539; 1257; 1233; 1172; 1012; 894; 719 & 690 cm⁻¹ |
| **15** | Spectre RMN ¹H (DMSO-d6, δ en ppm) : 7,60 (t large, J = 7,5 Hz, 2H) ; 7,70 (t large, J = 7,5 Hz, 1H) ; 7,82 (d, J = 2,0 Hz, 1H) ; 8,30 (d large, J = 8,5 Hz, 2H) ; 8,66 (s, 1H) ; 8,90 (d, J = 2,0 Hz, 1H). |
| **16** | Spectre RMN¹H (DMSO-d6, δ en ppm) : 7,13 (dd, J = 2,5 et 7,0 Hz, 1H) ; 7,57 (t large, J = 7,5 Hz, 2H) ; 7,68 (t large, J = 7,5 Hz, 1H) ; 7,92 (d, J = 2,5 Hz, 1H) ; 8,28 (d large, J = 8,0 Hz, 2H) ; 8,65 (m, 2H) Spectre de masse (IE) : m/z 256 (pic de base) : [M^{+.}], m/z 228 : [M^{+.}]-[CO], m/z 77 : Ph⁺ |
| **17** | Spectre RMN¹H (DMSO-d6, δ en ppm) : 7,51 (dd, J = 2,0 et 9,5 Hz, 1H) ; 7,58 (t large, J = 7,5 Hz, 2H) ; 7,68 (t large, J = 7,5 Hz, 1H) ; 7,71 (d, J = 9,5 Hz, 1H) ; 8,30 (d large, J = 8,0 Hz, 2H) ; 8,57 (s, 1H) ; 8,98 (s large, 1H). Spectre de masse (IE) : m/z 300 (pic de base) : [M^{+.}], m/z 272 : [M-CO]⁺, m/z 105 : PhCO⁺. Spectre IR (CCl₄) : 3152; 1651; 1528; 1261; 1233; 1055; 1010; 893; 713 & 686 cm⁻¹. |
| **18** | Spectre RMN ¹H (DMSO-d6, δ en ppm) : 7,39 (dd, J = 7,5 et 9,0 Hz, 1H) ; 7,47 (d large, J = 7,5 Hz, 1H) ; 7,59 (t large, J = 7,5 Hz, 2H) ; 7,70 (t large, J = 7,5 Hz, 1H) ; 7,82 (d large, J = 9,0 Hz, 1H) ; 8,33 (d large, J = 8,0 Hz, 2H) ; 8,48 (s, 1H) Spectre de masse (LCMS) : m/z 300 (pic de base) : [M+H]⁺. Spectre IR (KBr) : 3156; 1639; 1511; 1260; 1237; 1179; 1125; 895; 775; 705 & 697 cm⁻¹. |
| **19** | Spectre RMN ¹H (DMSO-d6, δ en ppm) : de 7,55 à 7,74 (m, 4H) ; 7,89 (d, J = 9,5 Hz, 1H) ; 8,29 (d large, J = 8,0 Hz, 2H) ; 8,68 (s, 1H) ; 9,40 (s large, 1H) Spectre de masse (IE) : m/z 247 (pic de base) : [M^{+.}], m/z 218 : [M-CO]⁺, m/z 192 : 219-[CO]. Spectre IR (KBr) : 3144; 2230; 1649; 1321; 1225; 1158; 1008; 906 & 729 cm⁻¹. |
| **20** | Spectre RMN ¹H (DMSO-d6, δ en ppm) : 6,04 (d, J = 7,5 Hz, 1H) ; 6,85 (s large, 2H) ; 6,93 (d, J = 9,0 Hz, 1H) ; 8,25 (dd, J = 7,5 et 9,0 Hz, 1H) ; 7,57 (t large, J = 7,5 Hz, 2H) ; 7,67 (t large, J = 7,5 Hz, 1H) ; 8,33 (d large, J = 8,0 Hz, 2H) ; 8,61 (s, 1H) Spectre de masse (IE) : m/z 237 (pic de base) : [M^{+.}], m/z 207 : [M-CO]⁺. Spectre IR (KBr) : 3372; 3202; 3129; 1637; 1548; 1497; 1232; 910; 733 & 697 cm⁻¹. |
| **21** | Spectre RMN ¹H (DMSO-d6, δ en ppm) : de 7,52 à 7,85 (m, 6H) ; 8,14 (s large, 1H) ; 8,31 (d large, J = 8,0 Hz, 2H) ; 8,73 (s, 1H) ; 9,20 (s large, 1H) Spectre de masse (IE) : m/z 265 (pic de base) : [M^{+.}], m/z 237 : [M-CO]⁺, m/z 77 : Ph⁺. Spectre IR (KBr) : 3428; 3190; 3144; 1682; 1626; 1483; 1394; 1275; 1241; 1011; 893; 733 & 521 cm⁻¹. |
| **22** | Spectre RMN ¹H (DMSO-d6, δ en ppm) : de 7,57 à 7,66 (m, 3H) ; 7,73 (t, J = 7,5 Hz, 1H) ; 7,80 (d, J = 9,5 Hz, 1H) ; 8,21 (d, J = 8,0 Hz, 2H) ; 8,66 (s, 1H) ; 9,09 (s, 1H) (absorptions larges) Spectre de masse (IE) : m/z 348 (pic de base) : [M^{+.}], m/z 320 : [M-CO]⁺, m/z 271: [M^{+.}]- Ph. Spectre IR (KBr) : 3054; 1660; 1648; 1593; 1357; 1268; 1226; 1012; 911; 723 & 598 cm⁻¹. |
| **23** | Spectre RMN ¹H (DMSO-d6, δ en ppm) : 6,67 (dd, J = 2,5 et 7,5 Hz, 1H) ; 6,75 (d, J = 2,5 Hz, 1H) ; 7,55 (t large, J = 7,5 Hz, 2H) ; 7,65 (t large, J = 7,5 Hz, 1H) ; 8,26 (d large, J = 8,0 Hz, 2H) ; 8,40 (s, 1H) ; 8,43 (d, J = 7,5 Hz, 1H) ; 10,5 (s, 1H) Spectre de masse (IE) : m/z 238 (pic de base) : [M⁺], m/z 210 : [M-CO]⁺, m/z 105 : PhCO⁺. Spectre IR (KBr) : 3165; 2597; 1637; 1551; 1234; 1160; 907; 714 & 698 cm⁻¹. |
| **24** | Spectre RMN ¹H (DMSO-d6, δ en ppm) : 7,59 (t large, J = 7,5 Hz, 2H) ; 7,70 (m, 2H) ; 8,29 (d large, J = 8,0 Hz, 2H) ; 8,74 (d, J = 3,0 Hz, 1H) ; 8,77 (m, 1H). Spectre de masse (IE) : m/z 258 (pic de base) : [M⁺], m/z 230 : [M-CO]⁺, m/z 239: [M⁺]-F. Spectre IR (KBr) : 3066; 3036; 2667; 1650; 1601; 1573; 1451; 1348; 1285; 1247; 1149; 914; 858 & 719 cm⁻¹. |
| **25** | Spectre RMN ¹H (DMSO-d6, δ en ppm) : 3,92 (s, 3H) ; 7,60 (t large, J = 7,5 Hz, 2H) ; 7,71 (t large, J = 7,5 Hz, 1H) ; 7,81 (m, 2H) ; 8,28 (d large, J = 8,0 Hz, 2H) ; 8,83 (s, 1H) ; 9,44 (m, 1H) Spectre de masse (IE) : m/z 280 (pic de base) : [M⁺], m/z 265 : [M⁺]-CH3 , m/z 252 : [M-CO]⁺, m/z 105 : PhCO⁺, m/z 77 : Ph⁺. Spectre IR (KBr) : 3063; 2750; 1730; 1654; 1577; 1435; 1322; 1215; 1103; 1010; 910; 766; 719 & 682 cm⁻¹. |
| **26** | Spectre RMN ¹H (DMSO-d6, δ en ppm) : 0,85 (t, J = 7,5 Hz, 3H) ; 1,12 (t, J = 7,0 Hz, 3H) ; de 1,59 à 1,85 (m, 2H) ; 3,37 (q partiellement masqué, J = 7,0 Hz, 2H) ; 4,26 (t, J = 7,0 Hz, 1H) ; 7,33 (dd, J = 1,5 et 9,5 Hz, 1H) ; 7,57 (t large, J = 7,5 Hz, 2H) ; de 7,64 à 7,74 (m, 2H) ; 8,32 (d large, J = 8,0 Hz, 2H) ; 8,55 (s large, 1H) ; 8,63 (s, 1H). Spectre de masse (IE) : m/z=308 [M]⁺, m/z=279 [M - C₂H₅]⁺ (pic de base), m/z=251 [m/z=279 - C₂H₄]⁺, m/z=77 [C₆H₅]⁺. |
| **27** | Spectre RMN ¹H (DMSO-d6, δ en ppm) : 2,62 (s, 3H) ; 7,59 (t, J = 7,5 Hz, 2H) ; 7,70 (t, J = 7,5 Hz, 1H) ; de 7,74 à 7,80 (m, 2H) ; 8,31 (d, J = 8,0 Hz, 2H) ; 8,72 (s, 1H) ; 9,51 (s large, 1H). Spectre IR (KBr) : 1679; 1641; 1598; 1575; 1483; 1384; 1290; 1255; 1209; 893 & 722 cm⁻¹. Spectre de masse (IE) : m/z=264 [M]⁺, m/z=221 [M - COCH₃]⁺, m/z=105 [C₇H₅O]⁺, m/z=77 [C₆H₅]⁺ (pic de base), m/z=43 CH₃CO⁺. |
| **28** | Spectre RMN ¹H (DMSO-d6, δ en ppm) : 7,59 (t large, J = 7,5 Hz, 2H) ; de 7,66 à 7,74 (m, 2H) ; 7,82 (d, J = 9,5 Hz, 1H) ; 8,30 (d large, J = 8,0 Hz, 2H) ; 8,83 (s, 1H) ; 9,38 (s large, 1H); 10,0 (s, 1H). Spectre IR (KBr) : 1694; 1645; 1288; 1229; 722 & 693 cm⁻¹ Spectre de masse : (IE) m/z=250 [M]⁺ m/z=221 [M - CHO]⁺ m/z=77 [C₆H₅]⁺ (pic de base). |
| **29** | Spectre RMN ¹H (DMSO-d6, δ en ppm) : 2,42 (s, 3H) ; de 7,40 à 7,52 (m, 3H) ; 7,78 (d, J = 9,5 Hz, 1H) ; 8,04 (s large, 1H) ; 8,13 (d large, J = 7,5 Hz, 1H) ; 8,56 (s, 1H) ; 8,89 (d, J = 2,5 Hz, 1H) Spectre de masse (IE) : m/z 270 : [M⁺] pic de base). |
| **30** | Spectre RMN ¹H (DMSO-d6, δ en ppm) : 2,42 (s, 3H) ; 7,39 (d large, J = 8,5 Hz, 2H) ; 7,43 (d, J = 2,0 et 10,0 Hz, 1H) ; 7,79 (d, J = 10,0 Hz, 1H) ; 8,26 (d large, J = 8,5 Hz, 2H) ; 8,56 (s, 1H) ; 8,90 (d, J = 2,0 Hz, 1H). Spectre de masse (IE) : m/z 270 (pic de base) : [M⁺], m/z 241 : [M-CO]⁺. |
| **31** | Spectre RMN ¹H (DMSO-d6, δ en ppm) : 7,45 (d, J = 2,0 et 10,0 Hz, 1H) ; 7,54 (m, 1H) ; 7,65 (m, 1H) ; 7,80 (d, J = 10,0 Hz, 1H) ; de 8,11 à 8,20 (m, 2H) ; 8,63 (s, 1H) ; 8,90 (d, J = 2,0 Hz, 1H) Spectre de masse (IE) : m/z 274 (pic de base) : [M⁺], m/z 246 : [M-CO]⁺. |
| **32** | Spectre RMN ¹H (DMSO-d6, δ en ppm) : 7,42 (t large, J = 8,5 Hz, 2H) ; 7,45 (dd, J = 2,0 et 10,0 Hz, 1H) ; 7,78 (d, J =10,0 Hz, 1H) ; 8,48 (dd large, J = 5,5 et 8,5 Hz, 1H) ; 8,60 (s, 1H) ; 8,90 (d, J = 2,0 Hz, 1H); Spectre de masse (IE) : m/z 274 (pic de base) : [M⁺], m/z 246 : [M-CO]⁺. |
| **33** | Spectre RMN ¹H (DMSO-d6, δ en ppm) : 7,47 (dd, J = 2,0 et 10,0 Hz, 1H) ; 7,61 (t large, J = 9,0 Hz, 1H) ; 7,81 (d, J = 10,0 Hz, 1H) ; 8,04 (d large, J = 9,0 Hz, 2H) ; 8,66 (s, 1H) ; 8,90 (s large, 1H). Spectre IR (KBr) : 3148; 2924; 1643; 1591; 1534; 1441; 1314; 1213; 1124; 1078; 990; 796 & 758 cm⁻¹. Spectre de masse (IE) : m/z 292 (pic de base) : [M⁺], m/z 264 : [M-CO]⁺, m/z 113 : COC₆H₃F₂. |
| **34** | Spectre RMN ¹H (DMSO-d6, δ en ppm) : 2,68 (s, 3H) ; 6,91 (d large, J = 7,0 Hz, 1H) ; 7,36 (dd, J = 7,0 et 9,5 Hz, 1H) ; de 7,53 à 7,63 (m, 3H) ; 7,68 (t large, J = 7,5 Hz, 1H) ; 8,35 (d large, J = 8,0 Hz, 2H) ; 8,50 (s, 1H) Spectre de masse (IE) : m/z 236 : [M+.] (pic de base) , m/z 207 : [M-CO]⁺. Spectre IR (KBr) : 2923; 1634; 1597; 1545; 1278; 1239; 1181; 897; 783; 700 & 470 cm⁻¹. |
| **35** | Spectre RMN ¹H (DMSO-d6, δ en ppm) : 2,82 (s, 3H) ; de 7,54 à 7,62 (m, 4H) ; 7,68 (t large, J = 7,5 Hz, 1H) ; 8,32 (d large, J = 8,0 Hz, 2H) ; 8,61 (s, 1H). Spectre de masse (IE) : m/z 314 (pic de base) : [M⁺], m/z 285 : [M-CO]⁺, m/z 235 : [M-Br]⁺. |
| **36** | Spectre RMN ¹H (DMSO-d6, δ en ppm) : 7,46 (dd, J = 2,0 et 9,5 Hz, 1H) ; 7,63 (t, J = 8,0 Hz, 1H) ; 7,76 (d large, J = 8,0 Hz, 1H) ; 7,81 (d, J = 9,5 Hz, 1H) ; 8,26 (d large, J = 8,0 Hz, 1H) ; 8,36 (t, J = 2,0 Hz, 1H) ; 8,63 (s, 1H) ; 8,91 (d large, J = 2,0 Hz, 1H). Spectre de masse (IC) : m/z 290 , [M⁺] |
| **37** | Spectre RMN ¹H (DMSO-d6, δ en ppm) : 7,47 (dd, J = 2,0 et 9,5 Hz, 1H) ; 7,67 (td, J = 8,0 et 10,5 Hz, 1H) ; 7,81 (d, J = 9,5 Hz, 1H) ; 8,31 (m, 1H) ; 8,46 (m, 1H) ; 8,64 (s, 1H) ; 8,91 (d large, J = 2,0 Hz, 1H). Spectre de masse (IC) : m/z 292 , [M⁺] |
| **38** | Spectre RMN ¹H (DMSO-d6, δ en ppm) : 2,95 (s, 6H) ; 7,64 (t, J = 7,5 Hz, 2H) ; 7,68 (d, J = 9,5 Hz, 1H) ; 7,76 (m, 2H) ; 7,99 (s large, 1H) ; 8,14 (d, J = 7,5 Hz, 2H) ; 8,67 (s, 1H). Spectre de masse (IE) : m/z 265 , [M⁺] (pic de base). |
| **39** | Spectre RMN ¹H (DMSO-d6, δ en ppm) : 4,60 (d, J = 5,5 Hz, 2H) ; 5,29 (t large, J = 5,5 Hz, 1H) ; 7,38 (d large, J = 8,0 Hz, 1H) ; 7,48 (t, J = 7,5 Hz, 1H) ; de 7,53 à 7,63 (m, 3H) ; de 7,65 à 7,76 (m, 3H) ; 7,81 (d large, J = 8,0 Hz, 1H) ; 8,34 (d large, J = 8,0 Hz, 2H) ; 8,65 (s, 1H) ; 9,00 (s large, 1H). Spectre IR (KBr) : 3386; 1645; 1600; 1542; 1490; 1264; 1224; 901; 781; 719 & 691 cm⁻¹. Spectre de masse (ES) : m/z=329 [M+H]⁺ (pic de base). |
| **40** | Spectre RMN ¹H (DMSO-d6, δ en ppm) : 4,57 (d, J = 5,5 Hz, 2H) ; 5,25 (t large, J = 5,5 Hz, 1H) ; 7,47 (d, J = 8,5 Hz, 2H) ; 7,59 (t large, J = 7,5 Hz, 2H) ; de 7,65 à 7,72 (m, 3H) ; 7,75 (dd, J = 2,0 et 9,5 Hz, 1H) ; 7,81 (d large, J = 9,5 Hz, 1H) ; 8,34 (d large, J = 8,0 Hz, 2H) ; 8,63 (s, 1H) ; 8,99 (s large, 1H). Spectre IR (KBr) : 3377; 1642; 1635; 1596; 1542; 1512; 1258; 1206; 1037; 1022; 1005; 896; 796 & 722 cm⁻¹ Spectre de masse (ES) : m/z=329 [M+H]⁺ (pic de base) |
| **41** | Spectre RMN ¹H (DMSO-d6, δ en ppm) : 4,48 (d, J = 5,5 Hz, 2H) ; 5,23 (t large, J = 5,5 Hz, 1H) ; de 7,32 à 7,50 (m, 4H) ; de 7,55 à 7,63 (m, 3H) ; 7,69 (t large, J = 7,5 Hz, 1H) ; 7,76 (d, J = 9,5 Hz, 1H) ; 8,34 (d large, J = 8,0 Hz, 2H) ; 8,64 (s, 1H) ; 8,66 (s large, 1H). Spectre IR (KBr) : 3407; 1644; 1543; 1266; 1018; 756 & 720 cm⁻¹ Spectre de masse (ES) : m/z=329 [M+H]⁺ (pic de base). |
| **42** | Spectre RMN ¹H (DMSO-d6, δ en ppm) : 7,60 (t large, J = 7,5 Hz, 2H) ; 7,69 (t large, J = 7,5 Hz, 1H) ; 7,78 (t, J = 7,5 Hz, 1H) ; de 7,81 à 7,88 (m, 2H) ; 7,98 (d large, J = 7,5 Hz, 1H) ; 8,10 (d large, J = 8,0 Hz, 1H) ; 8,28 (s large, 1H) ; 8,34 (d large, J = 8,0 Hz, 2H) ; 8,66 (s, 1H) ; 9,13 (s large, 1H) ; 10,1 (s, 1H). Spectre IR (KBr) : 3162; 2829; 2743; 1695; 1642; 1629; 1598; 1542; 1485; 1268; 1224; 1010; 894; 793; 707 & 690 cm⁻¹ Spectre de masse (IE) : m/z 326 [M⁺]. |
| **43** | Spectre RMN ¹H (DMSO-d6, δ en ppm) : 2,34 (s, 3H) ; 2,62 (s, 3H) ; 7,28 (d, J = 9,5 Hz, 1H) ; 7,53 (d, J = 9,5 Hz, 1H) ; 7,57 (t large, J = 7,5 Hz, 2H) ; 7,67 (t large, J = 7,5 Hz, 1H) ; 8,35 (d large, J = 8,0 Hz, 2H) ; 8,47 (s, 1H). Spectre de masse (IE) : m/z 250 (pic de base) : [M⁺], m/z 221 : [M-CO]⁺. |

Les composés selon l'invention ont fait l'objet d'essais phannacologiques permettant de déterminer leur effet modulateur sur NOT.

### Evaluation de l'activité in vitro sur cellules N2A

Des essais ont consisté à mesurer l'activité *in vitro* des composés de l'invention sur une lignée cellulaire (N2A) exprimant de manière endogène le récepteur de souris Nurr1 et transfectée de manière stable avec l'élément de réponse liant NOT (NBRE) couplé au gène rapporteur luciférase. Les EC₅₀ sont comprises entre 0,01 et 1000 nM. Les essais ont été réalisés selon le mode opératoire décrit ci dessous.
La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4.5 g/L de glucose et 0.4 mg/ml de Généticine. Après une semaine de culture les cellules sont récupérées par de la trypsine 0.25% pendant 30 secondes puis remises en suspension dans du DMEM sans rouge de phenol contenant 4.5g/L de glucose, 10% de sérum délipidé Hyclone et déposées dans des plaques blanches 96 puits fond transparent. Les cellules sont déposées à raison de 60.000 par puit dans 75 µL pendant 24 heures avant l'addition des produits. Les produits sont appliqués dans 25µL et incubés 24 heures supplémentaires. Le jour de la mesure, on ajoute à chaque puit un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻²M, puis dilués dans 100% de DMSO. Chaque concentration de produit est préalablement diluée dans du milieu de culture avant incubation avec les cellules contenant ainsi 0.625% final de DMSO.
Par exemple, les composés n° 1, 3 et 6 ont montré une EC₅₀ de respectivement 0,3 nM, 0,2 nM et 0,02 nM.

### Evaluation de la liaison au récepteur humain NOT

La liaison directe entre des composés de l'invention et le récepteur humain NOT a été évaluée en utilisant la technologie SPR (surface plasmon resonance). Dans cet essai la protéine est immobilisée de façon covalente à la matrice et la molécule à étudier est injectée dans la chambre contenant la sensor chip. Le signal est directement proportionnel à la quantité de produit fixé à la protéine. Les essais de liaison ont été réalisés dans un instrument BIACORE S51 (Biacore Inc., Piscataway N.J.). La protéine entière GST-NOT (NOT-FL) a été fournie par Invitrogen (PV3265). Le domaine de liaison au ligand de NOT (His-Thr-NOT 329-598) a été exprimé et purifié comme décrit dans Nature 423, 555-560. Les deux protéines, diluées à une concentration de 20µg/ml dans un tampon acétate pH 5.0 contenant 5 mM de DTT, ont été immobilisées sur une surface de carboxymethyl 5' dextrane (CM5 sensor chip, Biacore Inc.) par couplage amine en suivant le protocole recommandé par Biacore en éluant par un tampon HBS-N (10 mM HEPES, 0.15 M NaCl, 3 mM EDTA, pH 7.4). Approximativement 10000-15000 unités de resonance (RU) des protéines sont capturées sur la surface du sensor chip CM5. Les solutions stock des composés à étudier à 1,5 mM dans le DMSO sont diluées en série dans du tampon d'élution (50 mM HEPES pH8; 150 mM NaCl; 10 mM MgCl₂; 2% DMSO, 1 mM DTT) à des concentrations allant de 3,75 à 0,1 µM. Chaque concentration de produit est injectée à 4°C pendant 1 minute à 30 µl/min. La dissociation a été enregistrée pendant 5 minutes sans autre procédure de régénération de la surface. Les signaux obtenus sont corrigés en testant chaque concentration de produit sur une surface de dextrane non modifiée (blanc). Le signal dû au tampon de migration est déduit du signal total (« double referencing ») ainsi que l'effet du DMSO. L'analyse des signaux est effectuée à l'aide du logiciel d'analyse Biacore S51 (version 1.2.1). Les composés sont ensuite classés en fonction de leur niveau de fixation maximal et de paramètres cinétiques de liaison à la protéine immobilisée.
A titre d'exemple, les composés n° 1 et 3 ont une affinité moyenne.
Il apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.
Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto temporale, la dégénérescence corticobasale, la maladie de Pick), la sclérose en plaque ; les traumatismes cérébraux comme l'ischémie et les traumatismes crâniens et l'épilepsie ; les maladies psychiatriques comme la schizophrénie, la dépression, la dépendance à une substance, les troubles du déficit de l'attention et de l'hyperactivité ; les maladies inflammatoires comme les pathologies vasculaires, l'athérosclérose, les inflammations des articulations, l'arthrose, l'arthrite rhumatoïde ostéoarthrite, maladies inflammatoires allergiques telle que l'asthme et pour finir le traitement de l'ostéoporose, les cancers.

Ces composés pourraient être aussi utilisés comme traitement associé à des greffes et/ou transplantations de cellules souches.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

## Revendications

1. Composés répondant à la formule (I) : 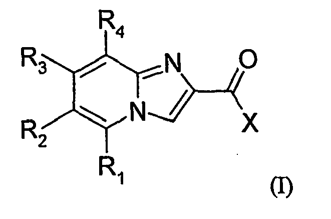 dans laquelle X représente :
. un groupe phényle éventuellement substitué par un ou plusieurs atomes ou groupes choisis indépendamment les uns des autres parmi halogène, (C₁-C₆)alcoxy, (C₁-C₆)alkyle, (C₃-C₇)cycloalkyle(C₁-C₆)alkyle, (C₃-C₇)cycloalkyle(C₁-C₆)alcoxy, hydroxy, amino, NRaRb ;
R₁ représente un atome d'hydrogène, un halogène, un groupe (C₁-C₆)alcoxy, un groupe (C₁-C₆)alkyle, un groupe (C₃-C₇)cycloalkyle(C₁-C₆)alkyle, un groupe (C₃-C₇)cycloalkyle(C₁-C₆)alcoxy, un hydroxy, un amino; le groupe (C₁-C₆)alkyle pouvant éventuellement être substitué par un ou plusieurs atomes ou groupes halogènes, hydroxy, amino, (C₁-C₆)alcoxy et le groupe (C₁-C₆)alcoxy pouvant éventuellement être substitué par un ou plusieurs atomes ou groupes halogène, hydroxy, amino, (C₁-C₆)alcoxy;
R₂ représente l'un des groupes suivants :
. un atome d'hydrogène,
. un groupe (C₁-C₆)alkyle éventuellement substitué par un ou plusieurs atomes ou groupes choisis indépendamment les uns des autres parmi halogène, hydroxy, amino, NRaRb,
. un groupe (C₁-C₆)alcoxy substitué par un ou plusieurs atomes ou groupes choisis indépendamment les uns des autres parmi halogène, hydroxy, amino, NRaRb,
. un groupe (C₃-C₇)cycloalkyle(C₁-C₆)alkyle,
. un groupe (C₃-C₇)cycloalkyle(C₁-C₆)alcoxy,
. un groupe (C₂-C₆)alcényle,
. un groupe (C₂-C₆)alcynyle,
. un groupe -CO-R₅
. un groupe -CO-NR₆R₇
. un groupe -CO-O-R₈
. un groupe -NR₉-CO-R₁₀
. un groupe -NR₁₁R₁₂
. un atome d'halogène,
. un groupe cyano,
. un groupe phényle éventuellement substitué par un ou plusieurs groupes choisis indépendamment les uns des autres parmi les atomes ou groupes halogène, (C₁-C₆)alcoxy, (C₁-C₆)alkyle, (C₃-C₇)cycloalkyle(C₁-C₆)alkyle, (C₃-C₇)cycloalkyle(C₁-C₆)alcoxy, hydroxy, amino, NRaRb, CO-R₅, le groupe (C₁-C₆)alkyle étant éventuellement substitué par un groupe hydroxy,
R₃ représente un atome d'hydrogène, un groupe (C₁-C₆)alkyle, un atome d'halogène, ou un groupe hydroxy,
R₄ représente un atome d'hydrogène ou un atome d'halogène,
R₅ représente un atome d'hydrogène ou un groupe (C₁-C₆)alkyle,
R₆ et R₇, identiques ou différents, représentent un atome d'hydrogène ou un groupe (C₁-C₆)alkyle ou forment avec l'atome d'azote un cycle de 4 à 7 chaînons incluant éventuellement un autre hétéroatome choisi parmi N, O ou S,
R₈ représente un groupe (C₁-C₆)alkyle,
R₉ et R₁₀, identiques ou différents, représentent un atome d'hydrogène ou un groupe (C₁-C₆)alkyle, R₁₁ représente un groupe (C₁-C₆)alkyle,
R₁₂ représente un hydrogène ou représente un groupe (C₁-C₆)alkyle,
R₁₁ et R₁₂ peuvent former avec l'atome d'azote un cycle de 4 à 7 chaînons incluant éventuellement un autre hétéroatome choisi parmi N,O ou S,
Ra représente un (C₁-C₆)alkyle
Rb représente un hydrogène ou un (C₁-C₆)alkyle
l'un au moins des substituants R₁, R₂, R₃ et R₄ n'est pas un hydrogène
à l'exception du composé où X est un phényle, R₃ est méthyle et R₁, R₂ et R₄ sont des hydrogènes ; du composé où X est un phényle, R₂ est chlore ou méthoxy et R₁, R₃ et R₄ sont des hydrogènes ; du composé où X est un p-tolyle, R₂ est un méthyle et R₁, R₃ et R₄ sont des hydrogènes ; du composé où X est un p-chlorophényle, R₁ est un chlore ou un méthoxy ou un méthyle, et R₂, R₃ et R₄ sont des hydrogènes ; du composé où X est un p-chlorophényle, R₂ est un chlore et R₁, R₃ et R₄ sont des hydrogènes ; du composé où X est un p-chlorophényle, R₂ est un méthyle et R₁, R₃ et R₄ sont des hydrogènes ;; du composé où X est un p-chlorophényle, R₄ est un méthyle, et R₁, R₂ et R₃ sont des hydrogènes ; du composé où X est un p-chlorophényle, R₃ est un méthyle et R₁, R₂ et R₄ sont des hydrogènes ; et du composé où X est un p-chlorophényle, R₁ et R₃ sont des méthyles et R₂ et R₄ sont des hydrogènes,
à l'état de base ou de sel d'addition à un acide.

2. Composé de formule (I) selon la revendication 1, **caractérisé en ce que** les composés de formule (I) pour lesquels :
R₁, R₃ et R₄ sont des atomes d'hydrogène
à l'état de base ou de sel d'addition à un acide.

3. Composé de formule (I) selon la revendication 1, **caractérisé en ce que** les composés de formule (I) pour lesquels : X est un groupe phényle, à l'état de base ou de sel d'addition à un acide.

4. Composés selon la revendication 1, choisis parmi :
(6-méthylimidazo[1,2-α]pyridin-2-yl)(phényl)méthanone et son Chlorhydrate (1:1)
Phényl[6-(trifluorométhyl)imidazo[1,2-α]pyridin-2-yl]méthanone Chlorhydrate (1:1)
Phényl[6-(trifluorométhyl)imidazo[1,2-α]pyridin-2-yl]méthanone
Phényl[6-(trifluorométhyl)imidazo[1,2-α]pyridin-2-yl]méthanone bromhydrate (1 : 1)
[6-(1-hydroxy-1-méthyléthyl)imidazo[1,2-α]pyridin-2-yl](phényl)méthanone et son bromhydrate (1 :1)
[6-(Hydroxyméthyl)imidazo[1,2-α]pyridin-2-yl](phényl)méthanone
(6-Chloroimidazo[1,2-α]pyridin-2-yl)(2-méthylphényl)méthanone
Phényl(6-phénylimidazo[1,2-α]pyridin-2-yl)méthanone
[5-(2-Méthoxyéthoxy)imidazo[1,2-α]pyridin-2-yl](phényl)méthanone
*N*-(2-Benzoylimidazo[1,2-α]pyridin-6-yl)acétamide
(6-Isopropénylimidazo[1,2-α]pyridin-2-yl)(phényl)méthanone
2-Benzoyl-*N*,*N*-diméthylimidazo[1,2-α]pyridine-6-carboxamide
2-Benzoyl-*N-*méthylimidazo[1,2-α]pyridine-6-carboxamide
Phényl(6-vinylimidazo[1,2-α]pyridin-2-yl)méthanone
(6-Ethylimidazo[1,2-α]pyridin-2-yl)(phényl)méthanone
(6-Fluoroimidazo[1,2-α]pyridin-2-yl)(phényl)méthanone
(6,8-dichloroimidazo[1,2-α]pyridin-2-yl)(phényl)méthanone et son chlorhydrate (1 : 1)
(7-Chloroimidazo[1,2-α]pyridin-2-yl)(phényl)méthanone
(6-Bromoimidazo[1,2-α]pyridin-2-yl)(phényl)méthanone
(5-Bromoimidazo[1,2-α]pyridin-2-ylXphényl)méthanone
2-Benzoylimidazo[1,2-α]pyridine-6-carbonitrile
(5-Aminoimidazo[1,2-α]pyridin-2-yl)(phényl)méthanone
2-Benzoylimidazo[1,2-α]pyridine-6-carboxamide
(6-iodolimidazo[1,2-α]pyridin-2-yl)(phényl)méthanone et son bromhydrate (1:1)
(7-hydroxyimidazo[1,2-α]pyridin-2-yl)(phényl)méthanone et son bromhydrate (1 :1)
(6,8-difluoroimidazo[1,2-α]pyridin-2-ylxphényl)méthanone et son bromhydrate (1 :1)
2-benzoylimidazo[1,2-α]pyridine-6-carboxylate de méthyle et son bromhydrate (1 :1)
[6-(1-Ethoxypropyl)imidazo[1,2-α]pyridin-2-yl](phényl)méthanone
1-(2-Benzoylimidazo[1,2-α]pyridin-6-yl)éthanone
2-Benzoylimidazo[1,2-α]pyridine-6-carbaldéhyde
(6-Chloroimidazo[1,2-α]pyridin-2-yl)(3-méthylphényl)méthanone
(6-Chloroimidazo[1,2-α]pyridin-2-yl)(4-méthylphényl)méthanone
(6-Chloroimidazo[1,2-α]pyridin-2-yl)(3-fluorophényl)méthanone
(6-Chloroimidazo[1,2-α]pyridin-2-yl)(4-fluorophényl)méthanone
(6-Chloroimidazo[1,2-α]pyridin-2-yl)(3,5-difluorophényl)méthanone
(5-Méthylimidazo[1,2-α]pyridin-2-yl)(phényl)méthanone
(6-Bromo-5-méthylimidazo[1,2-α]pyridin-2-yl)(phényl)méthanone
(6-Chloroimidazo[1,2-α]pyridin-2-yl)(3-chlorophényl)méthanone
(6-Chloroimidazo[1,2-α]pyridin-2-yl)(3,4-difluorophényl)méthanone
[6-(diméthylamino)imidazo[1,2-α]pyridin-2-yl](phényl)méthanone et son hexafluorophosphate (1 : 1)
{6-[3-(Hydroxyméthyl)phényl]imidazo[1,2-α]pyridin-2-yl}(phényl)méthanone
{6-[4-(Hydroxyméthyl)phényl]imidazo[1,2-α]pyridin-2-yl}(phényl)méthanone
{6-[2-(Hydroxyméthyl)phényl]imidazo[1,2-α]pyridin-2-yl}(phényl)méthanone
3-(2-Benzoylimidazo[1,2-α]pyridin-6-yl)benzaldéhyde
(5,6-Diméthylimidazo[1,2-α]pyridin-2-yl)(phényl)méthanone.
ou un sel d'addition de ces composés à un acide pharmaceutiquement acceptable.

5. Médicament, **caractérisé en ce qu'**il comprend un composé de formule (I) selon l'une quelconque des revendications 1 à 4, ou un sel d'addition de ce composé à un acide pharmaceutiquement acceptable.

6. Composition pharmaceutique, **caractérisée en ce qu'**elle comprend un composé de formule (I) selon l'une quelconque des revendications 1 à 4, ou un sel pharmaceutiquement acceptable, de ce composé, ainsi qu'au moins un excipient pharmaceutiquement acceptable.

7. Utilisation d'un composé de formule (I) selon l'une quelconque des revendications 1 à 4 pour la préparation d'un médicament destiné au traitement et à la prévention des maladies neurodégénératives.

8. Utilisation d'un composé de formule (I) selon l'une quelconque des revendications 1 à 4 pour la préparation d'un médicament destiné au traitement et à la prévention de la sclérose en plaque, des traumatismes cérébraux et de l'épilepsie.

9. Utilisation d'un composé de formule (I) selon l'une quelconque des revendications 1 à 4 pour la préparation d'un médicament destiné au traitement et à la prévention des maladies psychiatriques.

10. Utilisation d'un composé de formule (I) selon l'une quelconque des revendications 1 à 4 pour la préparation d'un médicament destiné au traitement et à la prévention des maladies inflammatoires.

11. Utilisation d'un composé de formule (I) selon l'une quelconque des revendications 1 à 4 pour la préparation d'un médicament destiné au traitement et à la prévention de l'ostéoporose et les cancers.

12. Utilisation d'un composé de formule (I) selon l'une quelconque des revendications 1 à 4 pour la préparation d'un médicament destiné au traitement et à la prévention de la maladie de Parkinson, d'Alzheimer, des tauopathies.

13. Utilisation d'un composé de formule (I) selon l'une quelconque des revendications 1 à 4 pour la préparation d'un médicament destiné au traitement et à la prévention de la schizophrénie, la dépression, la dépendance à une substance, les troubles du déficit de l'attention et de l'hyperactivité.

## Claims

1. Compounds corresponding to the formula (I): 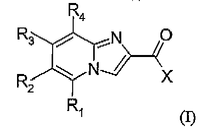 in which X represents:
.a phenyl group optionally substituted with one or more atoms or groups chosen, independently of each other, from halogen, (C₁-C₆)alkoxy, (C₁-C₆)alkyl, (C₃-C₇)cycloalkyl(C₁-C₆)alkyl, (C₃-C₇)cycloalkyl(C₁-C₆)alkoxy, hydroxyl, amino and NRaRb;
R₁ represents a hydrogen atom, a halogen, a group (C₁-C₆)alkoxy, a group (C₁-C₆)alkyl, a group (C₃-C₇)cycloalkyl(C₁-C₆)alkyl, a group (C₃-C₇)cycloalkyl(C₁-C₆)alkoxy, a hydroxyl or an amino; the group (C₁-C₆)alkyl possibly being substituted with one or more of the following atoms or groups: halogens, hydroxyl, amino, (C₁-C₆)alkoxy, and the group (C₁-C₆)alkoxy possibly being substituted with one or more of the following atoms or groups: halogen, hydroxyl, amino, (C₁-C₆)alkoxy;
R₂ represents one of the following groups:
. a hydrogen atom,
. a group (C₁-C₆)alkyl optionally substituted with one or more atoms or groups chosen, independently of each other, from halogen, hydroxyl, amino and NRaRb,
. a group (C₁-C₆)alkoxy substituted with one or more atoms or groups chosen, independently of each other, from halogen, hydroxyl, amino and NRaRb,
. a group (C₃-C₇)cycloalkyl(C₁-C₆)alkyl,
. a group (C₃-C₇)cycloalkyl(C₁-C₆)alkoxy,
. a group (C₂-C₆)alkenyl,
. a group (C₂-C₆)alkynyl,
. a group -CO-R₅,
. a group -CO-NR₆R₇,
. a group -CO-O-R₈,
. a group -NR₉-CO-R₁₀,
. a group -NR₁₁R₁₂,
. a halogen atom,
. a cyano group,
. a phenyl group optionally substituted with one or more groups chosen, independently of each other, from the following atoms or groups: halogen, (C₁-C₆)alkoxy, (C₁-C₆)alkyl, (C₃-C₇)cycloalkyl(C₁-C₆)alkyl, (C₃-C₇)cycloalkyl(C₁-C₆)alkoxy, hydroxyl, amino, NraRb, CO-R₅, the group (C₁-C₆)alkyl being optionally substituted with a hydroxyl group,
R₃ represents a hydrogen atom, a group (C₁-C₆)alkyl, a halogen atom or a hydroxyl group,
R₄ represents a hydrogen atom or a halogen atom,
R₅ represents a hydrogen atom or a group (C₁-C₆)alkyl,
R₆ and R₇, which may be identical or different, represent a hydrogen atom or a group (C₁-C₆)alkyl or form, with the nitrogen atom, a 4- to 7-membered ring optionally including another heteroatom chosen from N, O and S,
R₈ represents a group (C₁-C₆)alkyl,
R₉ and R₁₀, which may be identical or different, represent a hydrogen atom or a group (C₁-C₆)alkyl,
R₁₁ represents a group (C₁-C₆)alkyl,
R₁₂ represents a hydrogen or represents a group (C₁-C₆)alkyl,
R₁₁ and R₁₂ may form, with the nitrogen atom, a 4- to 7-membered ring optionally including another heteroatom chosen from N, O and S,
Ra represents a (C₁-C₆)alkyl,
Rb represents a hydrogen or a (C₁-C₆)alkyl,
at least one of the substituents R₁, R₂, R₃ and R₄ is not a hydrogen
with the exception of the compound for which X is a phenyl, R₃ is methyl and R₁, R₂ and R₄ are hydrogens; the compound for which X is a phenyl, R₂ is chlorine or methoxy and R₁, R₃ and R₄ are hydrogens; the compound for which X is a p-tolyl, R₂ is a methyl and R₁, R₃ and R₄ are hydrogens; the compound for which X is a p-chlorophenyl, R₁ is a chlorine, a methoxy or a methyl, and R₂, R₃ and R₄ are hydrogens; the compound for which X is a p-chlorophenyl, R₂ is a chlorine and R₁, R₃ and R₄ are hydrogens; the compound for which X is a p-chlorophenyl, R₂ is a methyl and R₁, R₃ and R₄ are hydrogens;; the compound for which X is a p-chlorophenyl,R₄ is a methyl, and R₁, R₂ and R₃ are hydrogens; the compound for which X is a p-chlorophenyl, R₃ is a methyl and R₁, R₂ and R₄ are hydrogen; and the compound for which X is a p-chlorophenyl, R₁ and R₃ are methyls and R₂ and R₄ are hydrogens,
in the form of the base or of an acid-addition salt.

2. Compound of formula (I) according to Claim 1, **characterized in that** the compounds of formula (I) for which:
R₁, R₃ and R₄ are hydrogen atoms
in the form of the base or of an acid-addition salt.

3. Compound of formula (I) according to Claim 1**, characterized in that** the compounds of formula (I) for which: X is a phenyl group, in the form of the base or of an acid-addition salt.

4. Compounds according to Claim 1, chosen from:
(6-Methylimidazo[1,2-α]pyridin-2-yl)(phenyl)methanone hydrochloride (1:1) Phenyl[6-(trifluoromethyl)imidazo[1,2-α]pyridin-2-yl]methanone hydrochloride (1:1)
Phenyl[6-(trifluoromethyl)imidazo[1,2-α]pyridin-2-yl]methanone
Phenyl[6-(trifluoromethyl)imidazo[1,2-α]pyridin-2-yl]methanone hydrobromide (1:1)
[6-(1-Hydroxy-1-methylethyl)imidazo[1,2-α]pyridin-2-yl](phenyl)methanone and
its hydrobromide (1:1)
[6-(Hydroxymethyl)imidazo[1,2-α]pyridin-2-yl](phenyl)methanone
(6-Chloroimidazo[1,2-α]pyridin-2-yl)(2-methylphenyl)methanone
Phenyl(6-phenylimidazo[1,2-α]pyridin-2-yl)methanone
[5-(2-Methoxyethoxy)imidazo[1,2-α]pyridin-2-yl](phenyl)methanone
*N*-(2-Benzoylimidazo[1,2-α]pyridin-6-yl)acetamide
(6-Isopropenylimidazo[1,2-α]pyridin-2-yl)(phenyl)methanone
2-Benzoyl-*N*,*N*-dimethylimidazo[1,2-α]pyridine-6-carboxamide
2-Benzoyl-*N*-methylimidazo[1,2-α]pyridine-6-carboxamide
Phenyl(6-vinylimidazo[1,2-α]pyridin-2-yl)methanone
(6-Ethylimidazo[1,2-α]pyridin-2-yl)(phenyl)methanone
(6-Fluoroimidazo[1,2-α]pyridin-2-yl)(phenyl)methanone
(6,8-bichloroimidazo[1,2-α]pyridin-2-yl)(phenyl)methanone and its hydrochloride (1:1)
(7-Chloroimidazo[1,2-α]pyridin-2-yl)(phenyl)methanone
(6-Bromoimidazo[1,2-α]pyridin-2-yl)(phenyl)methanone
(5-Bromoimidazo[1,2-α]pyridin-2-yl)(phenyl)methanone
2-Benzoylimidazo[1,2-α]pyridine-6-carbonitrile
(5-Aminoimidazo[1,2-α]pyridin-2-yl)(phenyl)methanone
2-Benzoylimidazo[1,2-α]pyridine-6-carboxamide
(6-Iodolimidazo[1,2-α]pyridin-2-yl)(phenyl)methanone and its hydrobromide (1:1) (7-Hydroxyimidazo[1,2-α]pyridin-2-yl)(phenyl)methanone and its hydrobromide (1:1)
(6,8-Difluoroimidazo[1,2-α]pyridin-2-yl)(phenyl)methanone and its hydrobromide (1:1)
2-benzoylimidazo[1,2-α]pyridine-6-carboxylate and its hydrobromide (1:1)
[6-(1-Ethoxypropyl)imidazo[1,2-α]pyridin-2-yl](phenyl)methanone
1-(2-Benzoylimidazo[1,2-α]pyridin-6-yl)ethanone
2-Benzoylimidazo[1,2-α]pyridine-6-carbaldehyde
(6-Chloroimidazo[1,2-α]pyridin-2-yl)(3-methylphenyl)methanone
(6-Chloroimidazo[1,2-α]pyridin-2-yl)(4-methylphenyl)methanone
(6-Chloroimidazo[1,2-α]pyridin-2-yl)(3-fluorophenyl)methanone
(6-Chloroimidazo[1,2-α]pyridin-2-yl)(4-fluorophenyl)methanone
(6-Chloroimidazo[1,2-α]pyridin-2-yl)(3,5-difluorophenyl)methanone
(5-Methylimidazo[1,2-α]pyridin-2-yl)(phenyl)methanone
(6-Bromo-5-methylimidazo[1,2-α]pyridin-2-yl)(phenyl)methanone
(6-Chloroimidazo[1,2-α]pyridin-2-yl)(3-chlorophenyl)methanone
(6-Chloroimidazo[1,2-α]pyridin-2-yl)(3,4-difluorophenyl)methanone
[6-(Dimethylamino)imidazo[1,2-α]pyridin-2-yl](phenyl)methanone and its
hexafluorophosphate (1:1)
{6-[3-(Hydroxymethyl)phenyl]imidazo[1,2-α]pyridin-2-yl}(phenyl)methanone
{6-[4-(Hydroxymethyl)phenyl]imidazo[1,2-α]pyridin-2-yl}(phenyl)methanone
{6-[2-(Hydroxymethyl)phenyl]imidazo[1,2-α]pyridin-2-yl}(phenyl)methanone
3-(2-Benzoylimidazo[1,2-α]pyridin-6-yl)benzaldehyde
(5,6-Dimethylimidazo[1,2-α]pyridin-2-yl)(phenyl)methanone,
or an addition salt of these compounds with a pharmaceutically acceptable acid.

5. Medicament, **characterized in that** it comprises a compound of formula (I) according to any one of Claims 1 to 4, or an addition salt of this compound with a pharmaceutically acceptable acid.

6. Pharmaceutical composition, **characterized in that** it comprises a compound of formula (I) according to any one of Claims 1 to 4, or a pharmaceutically acceptable salt of this compound, and also at least one pharmaceutically acceptable excipient.

7. Use of a compound of formula (I) according to any one of Claims 1 to 4, for the preparation of a medicament for preventing or treating neurodegenerative diseases.

8. Use of a compound of formula (I) according to any one of Claims 1 to 4, for the preparation of a medicament for preventing or treating multiple sclerosis, cerebral trauma and epilepsy.

9. Use of a compound of formula (I) according to anyone of Claims 1 to 4, for the preparation of a medicament for preventing or treating psychiatric diseases.

10. Use of a compound of formula (I) according to any one of Claims 1 to 4, for the preparation of a medicament for preventing or treating inflammatory diseases.

11. Use of a compound of formula (I) according to any one of Claims 1 to 4, for the preparation of a medicament for preventing or treating osteoporosis and cancers.

12. Use of a compound or formula (I) according to anyone of Claims 1 to 4, for the preparation of a medicament for preventing or treating Parkinson's disease, Alzheimer's disease and tauopathies.

13. Use of a compound of formula (I) according to any one of Claims 1 to 4, for the preparation of a medicament for preventing or treating schizophrenia, depression, substance dependency and attention-deficit hyperactivity disorder.

## Patentansprüche

1. Verbindungen der Formel (I): 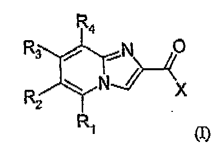 worin X für:
. eine Phenylgruppe, die gegebenenfalls durch ein oder mehrere Atome bzw. eine oder mehrere Gruppen, die unabhängig voneinander unter Halogen, (C₁-C₆)-Alkoxy, (C₁-C₆) -Alkyl, (C₃-C₇)-Cycloalkyl- (C₁-C₆) -alkyl, (C₃-C₇) -cycloalkyl- (C₁-C₆)-alkoxy, Hydroxy, Amino und NRaRb ausgewählt sind, substituiert ist, steht;
R₁ für ein Wasserstoffatom, ein Halogen, eine (C₁-C₆)-Alkoxygruppe, eine (C₁-C₆)-Alkylgruppe, eine (C₃-C₇) -Cycloalkyl- (C₁-C₆) -alkylgruppe, eine (C₃-C₇)-Cycloalkyl-(C₁-C₆)-alkoxygruppe, ein Hydroxy oder ein Amino steht; wobei die (C₁-C₆)-Alkylgruppe gegebenenfalls durch ein bzw. eine oder mehrere Halogenatome, Hydroxygruppen, Aminogruppen und/oder (C₁-C₆)-Alkoxygruppen substituiert ist und die (C₁-C₆)-Alkoxygruppe gegebenenfalls durch ein bzw. eine oder mehrere Halogenatome, Hydroxygruppen, Aminogruppen und/oder (C₁-C₆)-Alkoxygruppen substituiert ist;
R₂ für eine der folgenden Gruppen steht:
. ein Wasserstoffatom,
. eine (C₁-C₆) -Alkylgruppe, die gegebenenfalls durch ein oder mehrere Atome bzw. eine oder mehrere Gruppen, die unabhängig voneinander unter Halogen, Hydroxy, Amino und NRaRb ausgewählt sind, substituiert ist,
. eine (C₁-C₆)-Alkoxygruppe, die gegebenenfalls durch ein oder mehrere Atome bzw. eine oder mehrere Gruppen, die unabhängig voneinander unter Halogen, Hydroxy, Amino und NRaRb ausgewählt sind, substituiert ist,
. eine (C₃-C₇)-Cycloalkyl- (C₁-C₆)-alkylgruppe,
. eine (C₃-C₇)-Cycloalkyl-(C₁-C₆) -alkoxygruppe,
. eine (C₂-C₆)-Alkenylgruppe,
. eine (C₂-C₆)-Alkinylgruppe,
. eine -CO-R₅-Gruppe,
. eine -CO-NR₆R₇-Gruppe,
. eine -CO-O-R₈-Gruppe,
. eine -NR₉-CO-R₁₀-Gruppe,
. eine -NR₁₁R₁₂-Gruppe,
. ein Halogenatom,
. eine Cyanogruppe,
. eine Phenylgruppe, die gegebenenfalls durch eine oder mehrere Gruppen, die unabhängig voneinander unter Halogenatomen, (C₁-C₆)-Alkoxygruppen, (C₁-C₆)-Alkylgruppen, (C₃-C₇)-Cycloalkyl- (C₁-C₆) -alkylgruppen, (C₃-C₇) -Cycloalkyl-(C₁-C₆)-alkoxygruppen, Hydroxygruppen, Aminogruppen, NRaRb-Gruppen und CO-R₅-Gruppen ausgewählt sind, substituiert ist, wobei die (C₁-C₆)-Alkylgruppe gegebenenfalls durch eine Hydroxygruppe substituiert ist,
R₃ für ein Wasserstoffatom, eine (C₁-C₆)-Alkylgruppe, ein Halogenatom oder eine Hydroxygruppe steht,
R₄ für ein Wasserstoffatome oder ein Halogenatom steht,
R₅ für ein Wasserstoffatom oder eine (C₁-C₆)-Alkylgruppe steht,
R₆ und R₇ gleich oder verschieden sind und für ein Wasserstoffatom oder eine (C₁-C₆)-Alkylgruppe stehen oder mit dem Stickstoffatom einen 4- bis 7-gliedrigen Ring, der gegebenenfalls ein weiteres unter N, O und S ausgewähltes Heteroatom enthält, bilden,
R₈ für eine (C₁-C₆)-Alkylgruppe steht,
R₉ und R₁₀ gleich oder verschieden sind und für ein Wasserstoffatom oder eine (C₁-C₆) -Alkylgruppe stehen,
R₁₁ für eine (C₁-C₆)-Alkylgruppe steht,
R₁₂ für Wasserstoff oder eine (C₁-C₆)-Alkylgruppe steht,
R₁₁ und R₁₂ mit dem Stickstoffatom einen 4- bis 7-gliedrigen Ring, der gegebenenfalls ein weiteres unter N, O und S ausgewähltes Heteroatom enthält, bilden können,
Ra für (C₁-C₅) -Alkyl steht,
Rb für Wasserstoff oder (C₁-C₆)-Alkyl steht,
mindestens einer der Substituenten R₁, R₂, R₃ und R₄ nicht für Wasserstoff steht,
mit Ausnahme der Verbindung, in der X für Phenyl steht, R₃ für Methyl steht und R₁, R₂ und R₄ für Wasserstoffatome stehen; der Verbindung, in der X für Phenyl steht, R₂ für Chlor oder Methoxy steht und R₁, R₃ und R₄ für Wasserstoffatome stehen; der Verbindung, in der X für p-Tolyl steht, R₂ für Methyl steht und R₁, R₃ und R₄ für Wasserstoffatome stehen; der Verbindung, in der X für p-Chlorphenyl steht, R₁ für Chlor oder Methoxy oder Methyl steht und R₂, R₃ und R₄ für Wasserstoffatome stehen; der Verbindung, in der X für p-Chlorphenyl steht, R₂ für Chlor und R₁, R₃ und R₄ für Wasserstoffatome stehen; der Verbindung, in der X für p-chlorphenyl steht, R₂ für Methyl steht und R₁, R₃ und R₄ für Wasserstoffatome stehen; der Verbindung, in der X für p-Chlorphenyl steht, R₄ für Methyl steht und R₁, R₂ und R₃ für Wasserstoffatome stehen; der Verbindung, in der X für p-Chlorphenyl steht, R₃ für Methyl steht und R₁, R₂ und R₄ für Wasserstoffatome stehen; und der Verbindung, in der X für p-Chlorphenyl steht, R₁ und R₃ für Methylgruppen stehen und R₂ und R₄ für Wasserstoffatome stehen; in Basen- oder Säueradditionssalzform.

2. Verbindung der Formel (I) nach Anspruch 1, **dadurch gekennzeichnet, daß** die Verbindungen der Formel (I), für die:
R₁, R₃ und R₄ für Wasserstoffatome stehen,
in Basen- oder Säureadditionssalzform.

3. Verbindung der Formel (I) nach Anspruch 1, **dadurch gekennzeichnet, daß** die Verbindungen der Formel (I), für die: X für eine Phenylgruppe steht, in Basen- oder Säureadditionssalzform.

4. Verbindungen nach Anspruch 1, ausgewählt unter:
(6-Methylimidazo[1,2-*a*]pyridin-2-yl)(phenyl)-methanon und dessen Hydrochlorid (1:1)
Phenyl[6-(trifluormethyl)imidazo[1,2-*a*]pyridin-2-yl]methanon-hydrochlorid (1:1)
Phenyl [6-(trifluormethyl)imidazo[1,2-*a*]pyridin-2-yl]methanon
Phenyl [6-(trifluormethyl)imidazo[1,2-*a*] pyridin-2-yl]methanon-hydrobromid (1:1)
[6-(1-Rydroxy-1-methylethyl)imidazo[1,2-*a*]pyridin-2-yl](phenyl)methanon und dessen Hydrobromid (1:1)
[6-(Hydroxymethyl)imidazo[1,2-*a*]pyridin-2-yl]-(phenyl)methanon
(6-chlorimidazo[1,2-*a*]pyridin-2-yl) (2-methylphenyl)methanon
Phenyl(6-phenylimidazo[1,2-*a*]pyridin-2-yl)methanon
[5-(2-Methoxyethoxy)imidazo[1,2-*a*]pyridin-2-yl]-(phenyl)methanon
*N*-(2-Benzoylimidazo[1,2-*a*]pyridin-6-yl)acetamid (6-Isopropenylimidazo[1,2-*a*]pyridin-2-yl) (phenyl)-methanon
2-Benzoyl-*N,N*-dimethylimidazo[1,2-*a*]pyridin-6-carboxamid
2-Benzoyl-*N*-methylimidazo[1,2-*a*]pyridin-6-carboxamid
Phenyl(6-vinylimidazo[1,2-*a*]pyridin-2-yl)methanon
(6-Ethylimidazo[1,2-*a*]pyridin-2-yl)(phenyl)-methanon
(6-Fluorimidazo[1,2-*a*]pyridin-2-yl)(phenyl)-methanon
(6,8-Dichlorimidazo[1,2-*a*]pyridin-2-yl) (phenyl)-methanon und dessen Hydrochlorid (1:1)
(7-Chlorimidazo[1,2-*a*]pyridin-2-yl)(phenyl)-methanon
(6-Bromimidazo[1,2-*a*]pyridin-2-yl) (phenyl) methanon
(5-Bromimidazo[1,2-*a*]pyridin-2-yl)(phenyl)methanon
2-Benzoylimidazo[1,2-*a*]pyridin-6-carbonitril
(5-Aminoimidazo[1,2-*a*]pyridin-2-yl)(phenyl)-methanon
2-Benzoylimidazo[1,2-*a*]pyridin-6-carboxamid
(6-Iodimidazo[1,2-*a*]pyridin-2-yl) (phenyl)methanon und dessen Hydrobromid (1:1)
(7-Hydroxyimidazo[1,2-*a*]pyridin-2-yl)(phanyl)-methanon und dessen Hydrobromid (1:1)
(6, 8-Difluorimidazo [1,2-*a*]pyridin-2-yl) (phenyl)-methanone und dessen Hydrobromid (1:1)
2-Benzoylimidazo[1,2-*a*]pyridin-6-carbonsäuremethylester und dessen Hydrobromid (1:1)
[6-(1-Ethoxypropyl)imidazo[1,2-*a*]pyridin-2-yl]-(phenyl)methanon
1-(2-Eenzoylimidazo[1,2-*a*]pyxidin-6-yl)ethanon
2-Benzoylimidazo[1,2-*a*]pyridin-6-carbaldehyd
(6-Chlorimidazo[1,2-*a*]pyridin-2-yl) (3-methylphenyl)methanon
(6-Chlorimidazo[1,2-*a*]pyridin-2-yl)(4-methylphenyl)methanon
(6-Chlorimidazo[1,2-*a*]pyridin-2-yl)(3-fluorphenyl)methanon
(6-Chlorimidazo[1,2*-a*]pyridin-2-yl)(4-fluorphenyl)methanon
(6-Chlorimidazo[1,2*-a*]pyridin-2-yl)(3,5-difluorphenyl)methanon
(5-Methylimidazo[1,2*-a*]pyridin-2-yl)(phenyl)-methanon
(9-Brom-5-methylimidazo[1,2*-a*]pyridin-2-yl)-(phenyl)methanon
(5-Chlorimidazo[1,2*-a*]pyridin-2-yl)(3-chlorphenyl)methanon
(6-Chlorimidazo[1,2*-a*]pyridin-2-yl)(3,4-difluorphenyl)methanon
[6-(Dimethylamino)imidazo[1,2*-a*]pyridin-2-yl]-(phenyl)methanon und dessen Hexafluorophosphat (1:1)
{5-[3-(Hydroxymethyl)phenyl]imidazo[1,2*-a*]pyridin-2-yl}(phenyl)methanon
{6-[4-(Hydroxymethyl)phenyl]imidazo[1,2*-a*]pyridin-2-yl}(phenyl)methanon
{6-[2-(Hydroxymethyl)phenyl]imidazo[1,2*-a*]pyridin-2-yl}(phenyl)methanon
3-(2-Benzoylimidazo[1,2*-a*]pyridxn-6-yl)benzaldehyd (5,6-Dimethylimidazo[1,2*-a*]pyridin-2-yl)(phenyl)-methanon,
oder ein Additionssalz dieser Verbindungen mit einer pharmazeutisch unbedenklichen Säure.

5. Medikament, **dadurch gekennzeichnet, daß** es eine Verbindung der Formel (I) nach einem der Ansprüche 1 bis 4 oder ein Additionssalz dieser Verbindung mit einer pharmazeutisch unbedenklichen Säure enthält.

6. Pharmazeutische Zusammensetzung, **dadurch gekennzeichnet, daß** sie eine Verbindung der Formel (I) nach einem der Ansprüche 1 bis 4 oder ein pharmazeutisch unbedenkliches Salz dieser Verbindung sowie mindestens einen pharmazeutisch unbedenklichen Hilfsstoff enthält.

7. Verwendung einer Verbindung der Formel (I) nach einem der Ansprüche 1 bis 4 zur Herstellung eines Medikaments zur Behandlung und Prävention von neurodegenerativen Erkrankungen.

8. Verwendung einer Verbindung der Formel (I) nach einem der Ansprüche 1 bis 4 zur Herstellung eines Medikaments zur Behandlung und Prävention von Multipler Sklerose, Hirntraumen und Epilepsie.

9. Verwendung einer Verbindung der Formel (I) nach einem der Ansprüche 1 bis 4 zur Herstellung eines Medikaments zur Behandlung und Prävention von psychiatrischen Erkrankungen.

10. Verwendung einer Verbindung der Formel (I) nach einem der Ansprüche 1 bis 4 zur Herstellung eines Medikaments zur Behandlung und Prävention von entzündlichen Erkrankungen.

11. Verwendung einer Verbindung der Formel (I) nach einem der Ansprüche 1 bis 4 zur Herstellung eines Medikaments zur Behandlung und Prävention von Osteoporose und Krebserkrankungen.

12. Verwendung einer Verbindung der Formel (I) nach einem der Ansprüche 1 bis 4 zur Herstellung eines Medikaments zur Behandlung und Prävention von Parkinson-Krankheit, Alzheimer-Krankheit und Tauopathien.

13. Verwendung einer Verbindung der Formel (I) nach einem der Ansprüche 1 bis 4 zur Herstellung eines Medikaments zur Behandlung und Prävention von Schizophrenie, Depression, Substanzabhängigkeit und Aufmerksamkeitsdefizit-Hyperaktivitätssyndrom.
